# EUROPEAN PATENT APPLICATION

(11) **EP 4 582 808 A1**
(43) Date of publication of application: **09.07.2025**
(21) Application number: 23860345.0
(22) Date of filing: 29.08.2023
(51) Int. Cl.: G01N 33/543, G01N 33/545

(54) **IMMUNOASSAY METHOD, NON-SPECIFIC REACTION SUPPRESSION METHOD, IMMUNOASSAY REAGENT, IMMUNOASSAY REAGENT KIT, COMPOSITION, NON-SPECIFIC REACTION SUPPRESSING AGENT, AND USE**

(30) Priority: 29.08.2022 JP 2022136075
(71) Applicant: Sekisui Medical Co., Ltd., Tokyo 103-0027 (JP)
(72) Inventor: FUJIMURA Kengo, Tokyo 103-0027 (JP); SASAKI Ryuta, Tokyo 103-0027 (JP); IWASAKI Manami, Tokyo 103-0027 (JP)
(74) Representative: Ter Meer Steinmeister & Partner
(86) International application number: PCT/JP2023/031219
(87) International publication number: WO 2024/048583

(57) **Abstract**

An immunoassay method that is an immunologically measuring method of a target substance to be measured in a sample, the immunoassay method including performing an immunoreaction in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody. This immunoassay method enables suppression of a non-specific reaction that could not be resolved by non-specific reaction suppressing agents in the related art.

## Description

### TECHNICAL FIELD

The present invention relates to an immunoassay method, a non-specific reaction suppression method, an immunoassay reagent, an immunoassay reagent kit, a composition, a non-specific reaction suppressing agent, and a use.

Priority is claimed on Japanese Patent Application No. 2022-136075, filed August 29, 2022, the content of which is incorporated herein by reference.

### BACKGROUND ART

Examples of a measuring method in the field of diagnostic reagents include an immunoassay method of measuring a target substance to be measured present in a biological sample by using an immunoreaction. Since the immunoassay method uses an antigen-antibody reaction, the measuring method has very high specificity.

Various substances exist in the biological sample, and thus, binding that is not based on a specific reaction is promoted, or a specific immunoreaction is prevented, which causes a measurement error. Such a phenomenon is referred to as a non-specific reaction, and a substance that causes the non-specific reaction is referred to as a non-specific factor or the like.

As the non-specific factor, the existence of a heterophil antibody or a rheumatoid factor (RF) has been revealed. The heterophil antibody is a general term for human antibodies that exhibit reactivity with respect to animal-derived antibodies responsible for the main reaction of an immunoassay method, and human anti-mouse immunoglobulin antibody (HAMA) is known as a representative one. The rheumatoid factor has the same property as HAMA in that it appears in patients with rheumatoid arthritis and exhibits reactivity to animal-derived antibodies, and it is known that the actual entities of the rheumatoid factor are human immunoglobulin G and immunoglobulin M (Non Patent Document 1).

As a technology for suppressing a non-specific reaction in an immunoassay method, for example, Patent Document 1 and Patent Document 2 are known.

Patent Document 1 discloses a method of suppressing a non-specific reaction due to the RF by pretreating a sample with a sufficient amount of an animal-derived antibody having a binding ability to a reaction site (Fab) of the human rheumatoid factor (RF) in advance. Examples of such an animal-derived antibody include an anti-human immunoglobulin Fab antibody, an anti-human IgG antibody (Fab specific), an anti-human IgA antibody (Fab specific), and an anti-human IgM antibody (Fab specific).

Patent Document 2 discloses a method of suppressing a non-specific reaction by adding a polyclonal antibody against an IgM type natural antibody prepared from the same type of animal as the antibody used for measurement.

### Citation List

### Patent Documents

Patent Document 1: Japanese Unexamined Patent Application, First Publication No. H07-012818
Patent Document 2: Japanese Unexamined Patent Application, First Publication No. H11-287801

### Non Patent Document

Non Patent Document 1: Clinical Chemistry, Vol. 23, Supplement 175a-1 to 175a-10 (1994)

### SUMMARY OF INVENTION

### Technical Problem

The anti-human IgM polyclonal antibody, the anti-human IgG polyclonal antibody, and the anti-human IgA polyclonal antibody, which are used to suppress a non-specific reaction due to a natural antibody or RF, described in Patent Document 1, are currently non-specific reaction suppressing agent that have been put into practical use with various reagents. However, it has been found that there are still non-specific reactions that cannot be suppressed by this method.

In addition, the polyclonal antibody against the IgM type natural antibody described in Patent Document 2 needs to be prepared from the same type of animal as the antibody used for measurement, and there are restrictions on the preparation method and the antibody for measurement to be used.

An object of the present invention is to provide an immunoassay method using a new non-specific reaction suppressing agent that can suppress a non-specific reaction that cannot be resolved by non-specific reaction suppressing agents in the related art. Solution to Problem

In order to solve the above-described object, the present inventors have examined a non-specific reaction suppressing effect of various substances, and as a result, have found that a non-specific reaction can be suppressed by performing an immunoreaction in the presence of an anti-immunoglobulin L chain lambda monoclonal antibody and an anti-immunoglobulin L chain kappa monoclonal antibody, in the presence of an anti-immunoglobulin L chain lambda monoclonal antibody, or in the presence of an anti-immunoglobulin L chain kappa monoclonal antibody, thereby completing the present invention.

Specifically, the present invention has the following configurations.

[1] An immunoassay method that is an immunologically measuring method of a target substance to be measured in a sample, the immunoassay method including performing an immunoreaction in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody,
   provided that an anti-immunoglobulin L chain lambda antibody and an anti-immunoglobulin L chain kappa antibody is excluded from the target substance to be measured.
[2] The immunoassay method according to [1], in which the immunoassay method is an immunoassay method based on a homogeneous method or an immunoassay method based on a heterogeneous method.
[3] The immunoassay method according to [2], including a step of bringing a sample containing the target substance to be measured into contact with the anti-immunoglobulin L chain lambda monoclonal antibody or the anti-immunoglobulin L chain kappa monoclonal antibody, a step of adding a specific binding partner for the target substance to be measured, and a step of detecting a signal derived from a reaction between the target substance to be measured and the specific binding partner in the solution.
[4] The immunoassay method according to [2], in which the immunoassay method based on a homogeneous method is a latex turbidimetric immunoassay method.
[5] The immunoassay method according to [4], including a step of bringing a sample containing the target substance to be measured into contact with the anti-immunoglobulin L chain lambda monoclonal antibody or the anti-immunoglobulin L chain kappa monoclonal antibody, a step of adding latex particles carrying a specific binding partner for the target substance to be measured to the solution, and a step of optically detecting a degree of aggregation of the latex particles in the solution.
[6] A non-specific reaction suppression method in an immunologically measuring method of a target substance to be measured in a sample, the non-specific reaction suppression method including performing an immunoreaction in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody.
[7] The non-specific reaction suppression method according to [6], in which the immunologically measuring method is an immunoassay method based on a homogeneous method or an immunoassay method based on a heterogeneous method.
[8] The non-specific reaction suppression method according to [7], in which the immunoassay method based on a homogeneous method is a latex turbidimetric immunoassay method.
[9] The non-specific reaction suppression method according to [8], including a step of bringing a sample containing the target substance to be measured into contact with the anti-immunoglobulin L chain lambda monoclonal antibody or the anti-immunoglobulin L chain kappa monoclonal antibody in a solution, a step of adding latex particles carrying a specific binding partner for the target substance to be measured to the solution, and a step of optically detecting a degree of aggregation of the latex particles in the solution.
[10] An immunoassay reagent containing both or either of an anti-immunoglobulin L chain lambda monoclonal antibody or an anti-immunoglobulin L chain kappa monoclonal antibody.
[11] The immunoassay reagent according to [10], in which the immunoassay reagent is for an immunoassay method based on a homogeneous method or an immunoassay method based on a heterogeneous method.
[12] The immunoassay reagent according to [11], in which the immunoassay method based on a homogeneous method is a latex turbidimetric immunoassay method.
[13] An immunoassay reagent kit containing both or either of an anti-immunoglobulin L chain lambda monoclonal antibody or an anti-immunoglobulin L chain kappa monoclonal antibody.
[14] The immunoassay reagent kit according to [13], in which the immunoassay reagent is for an immunoassay method based on a homogeneous method or an immunoassay method based on a heterogeneous method.
[15] The immunoassay reagent kit according to [14], in which the immunoassay method based on a homogeneous method is a latex turbidimetric immunoassay method.
[16] The immunoassay reagent kit according to [14], in which the method based on a homogeneous method is a latex turbidimetric immunoassay method, and the immunoassay reagent kit includes
   (1) a first reagent containing both or either of an anti-immunoglobulin L chain lambda monoclonal antibody or an anti-immunoglobulin L chain kappa monoclonal antibody, and
   (2) a second reagent containing latex particles carrying a specific binding partner for a target substance to be measured.
[17] A composition containing both or either of an anti-immunoglobulin L chain lambda monoclonal antibody or an anti-immunoglobulin L chain kappa monoclonal antibody.
[18] A non-specific reaction suppressing agent in an immunoassay method, containing, as an effective component, both or either of an anti-immunoglobulin L chain lambda monoclonal antibody or an anti-immunoglobulin L chain kappa monoclonal antibody.
[19] Use of the composition according to [17] as a non-specific reaction suppressing agent in an immunoassay method.

### Advantageous Effects of Invention

According to the present invention, it is possible to provide an immunoassay method using a new non-specific reaction suppressing agent that can suppress a non-specific reaction that cannot be resolved by non-specific reaction suppressing agents in the related art.

In the immunoassay method according to the present invention, it is possible to suppress a non-specific reaction which has not been suppressed even by a commercially available non-specific reaction suppressing agent, and to perform an accurate measurement.

### DESCRIPTION OF EMBODIMENTS

### <Immunoassay method>

The immunoassay method according to the present invention is a method that is an immunological measuring method of a target substance to be measured in a sample, in which an immunoreaction is performed in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody. In other words, the immunoassay method is an immunologically measuring method of a target substance to be measured in a sample using a specific binding partner other than an anti-immunoglobulin L chain lambda monoclonal antibody and an anti-immunoglobulin L chain kappa monoclonal antibody, an anti-immunoglobulin L chain lambda monoclonal antibody, or an anti-immunoglobulin L chain kappa monoclonal antibody, in the presence of the anti-immunoglobulin L chain lambda monoclonal antibody and the anti-immunoglobulin L chain kappa monoclonal antibody as the non-specific reaction suppressing agent, in the presence of the anti-immunoglobulin L chain lambda monoclonal antibody as the non-specific reaction suppressing agent, or in the presence of the anti-immunoglobulin L chain kappa monoclonal antibody as the non-specific reaction suppressing agent.

The immunoassay method is further roughly classified into a homogeneous method and a heterogeneous method.

The homogeneous method is a measuring method in which a binding reaction that proceeds with a target substance to be measured in a mixed solution (reaction solution) of a sample and a reagent solution is specifically detected without B/F (binding/non-binding) separation, and the heterogeneous method is a measuring method in which a main reaction is proceeded and detected after washing and removing excess components that have not been involved in the measurement reaction by a B/F separation operation.

The heterogeneous method has an issue in that the number of steps is large and the measurement takes a long time because the washing step is performed, but has an advantage in that the method is relatively less susceptible to the influence of a non-specific reaction substance. On the other hand, the homogeneous method has an issue in that the method is susceptible to the influence of a non-specific reaction because the washing step is not performed, but is a method that is widely required in the field of clinical diagnosis because the number of steps is small, the method is simple, and the time required for measurement is short.

Examples of the homogeneous method include a measuring method using an immunoagglutination method (TIA) and immunochromatography (lateral flow type, flow-through type). The TIA is a method of qualitatively or quantitatively detecting an analyte in a sample based on the degree of aggregation of an immune complex formed by the crosslinking action of the analyte (target substance to be measured) by a specific binding partner such as an antibody, and among these, a latex turbidimetric immunoassay method (hereinafter, may be referred to as LTIA) using latex particles as an insoluble carrier to amplify an aggregation signal is suitable for optical detection and is easy to automate, thereby being a highly versatile measuring method that is applied to various examination items.

Examples of the heterogeneous method include an ELISA method using a wellshaped plate, a chemiluminescence method, and the like.

The present invention can be used in any of the above immunoassay methods, but the homogeneous method, which is relatively susceptible to the influence of a non-specific reaction, is preferable because of being more expected to obtain the effect of the present invention.

### (Anti-immunoglobulin L chain lambda monoclonal antibody, anti-immunoglobulin L chain kappa monoclonal antibody)

In the present invention, an anti-immunoglobulin L chain lambda monoclonal antibody and/or an anti-immunoglobulin L chain kappa monoclonal antibody are used as an effective component that suppresses a non-specific reaction.

The anti-immunoglobulin L chain lambda monoclonal antibody is an antibody that reacts with a lambda chain of an immunoglobulin L chain, and may be an antibody against a free L chain (including a Bence Jones protein) lambda chain or may be any of antibodies against an L chain lambda chain in a non-free state.

The anti-immunoglobulin L chain kappa monoclonal antibody is an antibody that reacts with a kappa chain of an immunoglobulin L chain, and may be an antibody against a free L chain kappa chain or may be any of antibodies against an L chain kappa chain in a non-free state.

The free immunoglobulin L chain is a monomer, dimer, or the like of an immunoglobulin L chain that is not associated with an immunoglobulin H chain (heavy chain). The L chain of immunoglobulin is classified into two types of kappa chain and lambda chain due to the difference in antigenicity. In the present specification, in the following, the anti-immunoglobulin L chain lambda antibody and the anti-immunoglobulin L chain kappa antibody each may be referred to as an anti-L chain lambda antibody and an anti-L chain kappa antibody in some cases.

In addition, unless otherwise specified in the present specification, the anti-L chain lambda antibody and the anti-L chain kappa antibody are intended to include both a monoclonal antibody and a polyclonal antibody.

In addition, in the present specification, kappa may be represented by κ and lambda may be represented by λ.

In addition, as the anti-L chain λ monoclonal antibody and the anti-L chain κ monoclonal antibody used in the present invention, a functional fragment of an antibody having reactivity with an antigen, in addition to the entire antibody molecule, can be used.

The anti-L chain λ monoclonal antibody and the anti-L chain κ monoclonal antibody used in the present invention are monoclonal antibodies obtained by a method such as phage display, in addition to those obtained through an immune step in a general animal. In general, a fusion cell (hybridoma) having an autonomous proliferation ability is produced by fusing an antibody-producing cell with a cell line derived from a myeloma, only the fusion cell that produces an antibody having reactivity to a target antigen is sorted (screening), and the selected fusion cell is subjected to a singulation (cloning) to establish an antibody-producing hybridoma. A desired monoclonal antibody is obtained by purifying the antibody derived from the present cell or transplanting the antibody derived from the present cell into a mouse to collect ascites.

The monoclonal antibody may be an antibody having an amino acid sequence derived from different animal species, specifically, a chimeric antibody, a humanized antibody, a fully human antibody, or the like. In addition, examples of the functional fragment of an antibody include F(ab')₂, Fab', or a single-chain antibody (scFv), which is fragment having an antigen-antibody reaction activity, a variable domain of heavy chain of heavy chain antibody (VHH antibody), and a new antigen receptor (IgNAR) antibody. Functional fragments of these antibodies can be produced by treating the antibody obtained as described above with a proteolytic enzyme (for example, pepsin, papain, or the like) or by utilizing a gene recombination technology. In the present invention, unless otherwise specified, the anti-L chain λ monoclonal antibody and the anti-L chain κ monoclonal antibody may include a functional fragment.

As described above, the anti-L chain λ monoclonal antibody and the anti-L chain κ monoclonal antibody can be used in combination, and furthermore, another known non-specific reaction suppressing agent such as an anti-human IgM antibody can be combined with each of them or a combination thereof.

In the present specification, the phrases that an antibody "reacts" with an antigen and an antibody "recognizes" an antigen are used synonymously, but the present invention is not limited to these examples and need to be interpreted in the broadest sense. The confirmation of whether or not the antibody "reacts" with the antigen (compound) can be performed by an antigen-immobilized ELISA method, a competitive ELISA method, a sandwich ELISA method, or the like, or can be performed by a method (an SPR method) using the principle of surface plasmon resonance. The SPR method can be performed using a device, a sensor, and reagents, which are commercially available under the name of Biacore (registered trademark).

The anti-L chain λ antibody and the anti-L chain κ antibody used in the present invention can be produced by dissolving each of a commercially available humanderived immunoglobulin and the like as an antigen (immunogen) in a solvent such as phosphate-buffered saline and administering the solution to an animal to immunize. Immunization may be performed using an emulsion after adding an appropriate adjuvant to the solution as necessary. As the adjuvant, in addition to commonly used adjuvants such as a water-in-oil type emulsion, a water-in-oil-in-water type emulsion, an oil-in-water emulsion, a liposome, and an aluminum hydroxide gel, a protein or a peptide-like substance derived from a biological component may be used. For example, an incomplete freund's adjuvant or a complete freund's adjuvant can be suitably used. The route of administration, the dose, and the timing of administration of the adjuvant are not particularly limited, but is desirable to be appropriately selected such that a desired immune response can be enhanced in the animal that is immunized with the antigen.

The type of the animal used for the immunization is not particularly limited, but a mammal is preferable, and for example, a mouse, a rat, a cow, a rabbit, a goat, a sheep, an alpaca, or the like can be used, and the mouse or the rat can be more preferably used. The immunity of an animal may be performed according to a general method, and for example, the immunity can be performed by injecting a solution of an antigen, preferably a mixture of an antigen and an adjuvant, subcutaneously, intradermally, intravenously, or intraperitoneally into an animal. Since the immune response generally varies depending on the type and lineage of the immunized animal, it is desirable to appropriately set the immunization schedule according to the animal to be used. It is preferable that the administration of the antigen is repeated several times after the first immunization.

The following operations are performed to obtain a monoclonal antibody, but the present invention is not limited thereto. A method for producing a monoclonal antibody itself is well known and is commonly used in the art, and thus, those skilled in the art can easily produce the monoclonal antibody used in the immunological analysis method according to the present invention (for example, see Chapter 6 of Reference Document 1).

After the final immunization, spleen cells or lymph node cells, which are antibody-producing cells, are excised from the immunized animal, and fused with a cell line derived from a myeloma, which has high proliferation ability, thereby a hybridoma can be produced. It is preferable to use a cell having a high antibody production ability (quality and quantity) for cell fusion, and it is preferable that a cell line derived from a myeloma is compatible with an animal from which the antibody-producing cell to be fused is derived. The cell fusion can be performed according to a known method in the field of the technology to which the present invention belongs, and for example, a polyethylene glycol method, a method using Sendai virus, a method using current, or the like can be adopted. The obtained hybridoma can be proliferated under general conditions in the art, and a desired hybridoma can be selected while confirming the properties of the antibody to be produced. The cloning of the hybridoma can be performed, for example, by a well-known method such as a limiting dilution method or a soft agar method.

The selection of the hybridoma can also be efficiently performed at the stage of selection in consideration of the conditions under which the produced antibody is used for the actual measurement. For example, a hybridoma that produces a desired antibody can be more efficiently sorted by reacting an antibody obtained from an immunized animal with an antigen (L chain lambda chain or L chain kappa chain) immobilized on a solid phase and comparing the reactivity with a reactivity in the absence of the antigen as a control.

More specifically, a case where the antibody reacts with the lambda chains of both of two or more classes (for example, IgA, IgG, and the like) can be determined to be lambda chain-specific. Similarly, a case where the antibody reacts with the kappa chains of both of two or more classes (for example, IgA, IgG, and the like) is determined to be kappa chain-specific. On the other hand, in a case where the antibody reacts with one type of class (for example, IgA) regardless of the lambda chain or the kappa chain and does not react with a class (for example, IgG) different from the one type of class, the antibody is determined to be not specific to the kappa chain and the lambda chain, but to be specific to the one kind of class (IgA in the present example).

After the cloning step, the binding ability between the produced antibody and the L chain lambda chain or the L chain kappa chain is measured using a method such as an ELISA method, an RIA method, or a fluorescent antibody method, thereby it is possible to confirm whether or not the sorted hybridoma produces a monoclonal antibody having desired properties.

A monoclonal antibody having a desired characteristic can be produced by large-scale culturing the hybridoma sorted as described above. A method for large-scale culture is not particularly limited, and examples thereof include a method of culturing a hybridoma in an appropriate culture medium to produce a monoclonal antibody in the culture medium, a method of injecting a hybridoma intraperitoneally into a mammal to proliferate the hybridoma and produce an antibody in ascites. Purification of the monoclonal antibody can be performed by appropriately combining the above-described purification method for an antibody from an antiserum, for example, DEAE anion exchange chromatography, affinity chromatography, an ammonium sulfate fractionation method, a PEG fractionation method, an ethanol fractionation method, and the like.

In the present invention, examples of the method of allowing both or either of the anti-L chain λ monoclonal antibody and the anti-L chain κ monoclonal antibody to be present in the immunoreaction system include a method of using the antibody as one of the reagent components of an immunoassay reagent and a method of adding the antibody to a diluent liquid or a pretreatment liquid of a sample, and the like.

The term "within the immunoreaction system" refers to, for example, a liquid phase in which a sample and a liquid immunoassay reagent are mixed and an immunoreaction is performed in a case where the immunoassay reagent is a liquid reagent.

For example, in the case of the LTIA method, the sample may be mixed with an LTIA measurement reagent containing both or either of the anti-L chain λ monoclonal antibody and the anti-L chain κ monoclonal antibody, or the sample may be mixed with a pretreatment liquid containing both or either of the anti-L chain λ monoclonal antibody and the anti-L chain κ monoclonal antibody in advance and then mixed with an immunoassay reagent.

In addition, in the case of the ELISA method, the sample may be mixed with a pretreatment liquid containing both or either of an anti-L chain λ monoclonal antibody and an anti-L chain κ monoclonal antibody in advance and then added dropwise to a microplate, or the sample may be mixed with a solution containing both or either of an anti-L chain λ monoclonal antibody and an anti-L chain κ monoclonal antibody, and a detection antibody, and then added dropwise to a microplate.

In addition, in the case of the chemiluminescence method reagent, the sample may be mixed with the pretreatment liquid containing both or either of the anti-L chain λ monoclonal antibody and the anti-L chain κ monoclonal antibody in advance and then mixed with the immunoassay reagent, or the immunoassay reagent (for example, a solution containing a detection antibody or antigen, and magnetic particles) may contain both or either of the anti-L chain λ monoclonal antibody and the anti-L chain κ monoclonal antibody.

Furthermore, in a case where an immunoreaction is performed in a solid phase such as an immunochromatography method, the term "within the immunoreaction system" means a solid phase in which an immunoreaction of the liquid sample and the binding partner is performed. In this case, the sample may be mixed with a pretreatment liquid containing both or either of the anti-L chain λ monoclonal antibody and the anti-L chain κ monoclonal antibody in advance and then added dropwise to the immunochromatographic test piece, or both or either of the anti-L chain λ monoclonal antibody and the anti-L chain κ monoclonal antibody may be dried and held on the sample pad, and in a case where the sample is added dropwise thereto, both or either of the anti-L chain λ monoclonal antibody and the anti-L chain κ monoclonal antibody may be dissolved to develop a solid phase, thereby the antibody may be in a state to be present in the reaction system.

In the present invention, the concentration of the anti-L chain λ monoclonal antibody may be any concentration as long as it does not significantly affect the immunoreaction between the target substance to be measured and the specific binding partner and can exhibit a desired non-specific reaction suppressing effect, and it can be appropriately set by those skilled in the art according to the type of the target substance to be measured or the specimen.

The concentration of the anti-L chain λ monoclonal antibody in the immunoreaction system varies depending on the reagent composition of the immunoreaction system, and is 0.1 to 1,000 µg, preferably 1.0 to 750 µg, more preferably 2.0 to 500 µg, still more preferably 3.0 to 250 µg, and most preferably 5.0 to 150 µg, as the weight of the anti-L chain λ antibody with respect to 10 µL of the sample. However, the present invention is not limited these concentrations. For example, 5.0 to 120 µg, 5.0 to 110 µg, 5.0 to 100 µg, 5.0 to 80 µg, 5.0 to 60 µg, or 5.0 to 50 µg may be also preferable in some cases. In addition, the lower limit of the above range may be also preferably 10.0 µg or more, 15.0 µg or more, or 20.0 µg or more in some cases.

The concentration of the anti-L chain κ monoclonal antibody in the immunoreaction system varies depending on the reagent composition of the immunoreaction system, and is 0.1 to 1,000 µg, preferably 1.0 to 750 µg, more preferably 2.0 to 500 µg, still more preferably 3.0 to 250 µg, and most preferably 5.0 to 150 µg, as the weight of the anti-L chain κ antibody with respect to 10 µL of the sample. However, the present invention is not limited these concentrations. For example, 5.0 to 120 µg, 5.0 to 110 µg, 5.0 to 100 µg, 5.0 to 80 µg, 5.0 to 60 µg, or 5.0 to 50 µg may be also preferable in some cases. In addition, the lower limit of the above range may be also preferably 10.0 µg or more, 15.0 µg or more, or 20.0 µg or more in some cases.

In the present invention, the anti-L chain λ monoclonal antibody and the anti-L chain κ monoclonal antibody may be used alone, or a plurality of types of anti-L chain λ monoclonal antibodies, a plurality of types of anti-L chain κ monoclonal antibodies, or a mixture of the anti-L chain λ monoclonal antibody and the anti-L chain κ monoclonal antibody may be used.

In a case where the anti-L chain λ monoclonal antibody and the anti-L chain κ monoclonal antibody are mixed and used, the mixing ratio can be set to 20:1 to 1:20, preferably 10:1 to 1:10, more preferably 5:1 to 1:5, and most preferably 2:1 to 1:2, in terms of the weight of each antibody. The normal ratio of the κ chain to the λ chain in serum in healthy individuals is in a range of 0.26 to 1.65 (Reference Document 2). In addition, the normal ratio of the κ chain to the λ chain that is not free in serum is about 2.0. Those skilled in the art can appropriately select the mixing ratio according to the race, disease group, age, and the like of the specimen donor to be measured.

**In** addition, both or either of the anti-L chain λ monoclonal antibody and the anti-L chain κ monoclonal antibody in the free state, which are not bound to an insoluble carrier or the like may be used, or both or either of the anti-L chain λ monoclonal antibody and the anti-L chain κ monoclonal antibody that are bound to an insoluble carrier may be used.

In addition, in a case where a plurality of types of both or either of the anti-L chain λ monoclonal antibody and the anti-L chain κ monoclonal antibody are used in combination, the concentration of the plurality of antibodies is preferably within the above-described concentration range.

Furthermore, in a case where both or either of the anti-L chain λ monoclonal antibody and the anti-L chain κ monoclonal antibody are contained in advance in the measurement reagent according to the present invention, it is preferable that the antibodies are contained in advance in the measurement reagent to have the above-described concentration within the reaction system.

### (Latex turbidimetric immunoassay method (LTIA method))

The LTIA method which is one of the immunoassay methods according to the present invention will be described. The method of measuring a target substance to be measured by the LTIA method can be roughly classified into two types.

The first method is a method of reacting latex particles on which a specific binding partner for a target substance to be measured is immobilized, with the target substance to be measured to form a sandwich-type immune complex, and measuring the target substance to be measured from the degree of aggregation of the latex particles accompanying the formation of the immune complex.

The second method is a method of adding protein or the like on which a plurality of target substances to be measured or analogs thereof (including fragments thereof) are immobilized, to a reagent, allowing the target substance to be measured in the reagent and the target substance to be measured in a sample to compete with each other to inhibit the formation of an immune complex of the target substance to be measured in the reagent and latex particles in which a specific binding partner for the target substances to be measured is immobilized, and measuring the target substance to be measured (antigen) from the degree of aggregation inhibition of the latex particles accompanying the inhibition of the formation of the immune complex.

Any substance can be selected as the target substance to be measured and the specific binding partner for the target substance to be measured, depending on the purpose. For example, in a case where the target substance to be measured is an antigen, an antibody such as a polyclonal antibody or a monoclonal antibody (including a recombinant type antibody and a functional fragment of each antibody) can be selected as a specific binding partner for the target substance to be measured. In a case where the target substance to be measured is an antibody, an antigen such as a native type or a recombinant type antigen can be selected as a specific binding partner for the target substance to be measured.

The present invention can be used for any of the above methods, and specifically the following steps are exemplary examples.

(1) A step of bringing a sample containing a target substance to be measured into contact with an anti-L chain λ monoclonal antibody or an anti-L chain κ monoclonal antibody in a solution
(2) A step of adding a specific binding partner for the target substance to be measured after the step (1)
   or
(2') a step of adding latex particles carrying a specific binding partner for the target substance to be measured to the solution after the step (1)
(3) A step of detecting a signal derived from a reaction between the target substance to be measured and the specific binding partner in the solution after the step (2)
   or
(3') a step of optically detecting a degree of aggregation of the latex particles in the solution after the step (2')

Here, the steps (3) and (3') mean "a step of measuring an aggregation reaction between the target substance to be measured and the latex particles without performing a washing/separation step in the middle of the step (2) or after the step (2)".

In the LTIA method, the test substance can be measured by optically or electrochemically observing the degree of aggregation generated. Examples of the method of optically observing the sample include a method of measuring a scattered light intensity, an absorbance, or a transmitted light intensity with an optical device (endpoint method, rate method, or the like). A measurement value such as an absorbance obtained by measuring a sample is compared with a measurement value such as an absorbance obtained by measuring a standard substance (a sample in which a concentration of a target substance to be measured is known), and a concentration (quantitative value) of the target substance to be measured contained in the sample is calculated. The measurement of the absorbance or the like of transmitted light, scattered light, or the like may be a single-wavelength measurement or a dual-wavelength measurement (difference or ratio between two wavelengths). The measurement wavelength is generally selected from 500 nm to 900 nm.

The measurement of the target substance to be measured in the sample according to the present invention may be performed by a hand method or by using a device such as a measuring device. The measuring device may be a general-purpose automatic analysis device or a dedicated measuring device (dedicated machine). In addition, it is preferable that this measurement is carried out by a method in which a plurality of operation steps such as a two-step method (two-reagent method) are performed.

### (Latex particles carrying specific binding partner)

The specific binding partner for the target substance to be measured can be immobilized on and carried by the latex particles by a known method such as a physical adsorption method, a chemical bonding method, or a combination thereof. In a case of the physical adsorption method, according to a known method, the method can be carried out by mixing and bringing a specific binding partner for a target substance to be measured and latex particles into contact with each other in a solution such as a buffer solution, or bringing a specific binding partner for a target substance to be measured dissolved in a buffer solution or the like into contact with a carrier. In addition, in a case of carrying out the chemical bonding method, according to a known method described in "Clinical Pathology Extra Issue No. 53 Special Feature, Immunoassay on Clinical Examination - Techniques and Applications -" edited by the Japanese Society of Clinical Pathology, published by The Clinical Pathology Press in 1983; "New Biochemical Experimental Course 1 Protein IV" edited by the Japanese Biochemical Society, published by Tokyo Kagaku Dozin in 1991; and the like, the method can be performed by mixing and bringing a specific binding partner for a target substance to be measured and a carrier into contact with a divalent crosslinking reagent such as glutaraldehyde, carbodiimide, imide ester, or maleimide, and reacting each of an amino group, a carboxyl group, a thiol group, an aldehyde group, a hydroxyl group, or the like of the specific binding partner for a target substance to be measured and the carrier with the divalent crosslinking reagent.

The synthetic polymer constituting the latex particles according to the present invention is not particularly limited, and examples thereof include polystyrene, a styrene-styrene sulfonate copolymer, a methacrylic acid polymer, an acrylic acid polymer, an itaconic acid polymer, and a styrene-hydrophilic carboxymonomer copolymer: for example, a styrene-methacrylic acid copolymer, a styrene-acrylic acid copolymer, a styrene-itaconic acid copolymer, and the like. Among these, a styrene-methacrylic acid copolymer, a styrene-itaconic acid copolymer, styrene, or a styrene-styrene sulfonate copolymer is preferable. In particular, a styrene and a styrene-(meth)acrylic acid copolymer is preferable.

It is preferable that the specific binding partner for the specific substance carried by the latex particles has a plurality of types to form a sandwich. In a case where a plurality of antibody recognition sites are present in the specific substance, the specific binding partner may have one type. For example, in a case where the specific binding partner is a monoclonal antibody, a plurality of monoclonal antibodies having different recognition sites are used. In addition, for example, in a case where the specific binding partner is a polyclonal antibody, the specific binding partner may be a polyclonal antibody derived from one type of antiserum or may be a polyclonal antibody derived from a plurality of types of antisera. In addition, a monoclonal antibody and a polyclonal antibody may be used in combination.

In a case where it is necessary to perform a treatment to suppress natural aggregation of latex particles, a non-specific reaction, or the like, the treatment may be performed by a known method such as bringing proteins such as bovine serum albumin (BSA), casein, gelatin, egg white albumin, or salts thereof, surfactants, skimmed milk powder or the like into contact with and coating the surface of the latex particles to perform the blocking treatment (masking treatment) of the carrier.

### (Immunoassay reagent)

The immunoassay method according to the present invention is performed using an immunoassay reagent, and the immunoassay reagent contains both or either of the above-described anti-L chain λ monoclonal antibody or anti-L chain κ monoclonal antibody, in addition to a main component of an immunoreaction. The main component of the reaction includes a binding partner (other than the anti-L chain λ antibody and the anti-L chain κ antibody) that is specific to the target substance to be measured, and examples thereof include insoluble carriers such as immunoassay particle, an immunochromatographic test piece, and a microplate, and the like.

The immunoassay reagent according to the present invention may contain a buffer, a protein, a peptide, an amino acid, a nucleic acid, a lipid, a phospholipid, a sugar, an inorganic salt, a polymer compound, a surfactant, other non-specific reaction suppressing agent, a preservative, or the like, as long as the non-specific reaction suppressing effect is not impaired. As a component that buffers and adjusts the pH, ion strength, osmotic pressure, and the like of the sample, for example, a buffer solution such as acetic acid, citric acid, phosphoric acid, tris, glycine, boric acid, carbonic acid, phthalic acid, succinic acid, maleic acid, or imidazole, a Good's buffer solution, or a sodium salt, a potassium salt, a calcium salt, or the like of these may be contained. In addition, a polymer such as polyvinylpyrrolidone or a phospholipid polymer may be contained as a component that enhances the formation of aggregates.

The concentration of both or either of the anti-L chain λ monoclonal antibody and the anti-L chain κ monoclonal antibody in each of the constituent reagents may be included in a form that can be adjusted to the concentration in the immunoreaction system in a state of being mixed with a reagent and a sample at the time of measurement, and varies depending on each type of reagent.

### (Reagent kit)

The reagent kit according to the present invention is characterized in that the kit configuration includes at least both or either of an anti-L chain λ monoclonal antibody and an anti-L chain κ monoclonal antibody. Examples of the kit configuration include a sample diluent liquid and a sample extract liquid, in addition to a reagent involved in an immunoassay, and both or either of the anti-L chain λ monoclonal antibody and the anti-L chain κ monoclonal antibody according to the present invention can be included in any one or two or more of these. Examples of the configuration of the kit include a user manual, a sample collection tool (a collection pipette, a syringe, a cotton swab, a filtration filter, and the like) in addition to the above.

Hereinafter, each of the reagent configurations in which each immunoassay method is adopted will be described.

### <Latex turbidimetric immunoassay method>

Examples of a reagent (LTIA reagent) in a case where the immunoassay method is a latex turbidimetric immunoassay method are described.

(1) A first reagent containing both or either of anti-L chain λ monoclonal antibody or anti-L chain κ monoclonal antibody
(2) A second reagent containing latex particles carrying a specific binding partner for a target substance to be measured

The first reagent typically contains a buffer solution, and the concentration of both or either of the anti-L chain λ monoclonal antibody and the anti-L chain κ monoclonal antibody in the buffer solution may be included in a form that can be adjusted to the concentration of both or either of the preferred anti-L chain λ monoclonal antibody and anti-L chain κ monoclonal antibody in a state where the reagent and the sample are mixed at the time of measurement, and varies depending on each reagent type. Both or either of the anti-L chain λ monoclonal antibody and the anti-L chain κ monoclonal antibody may be included in the second reagent in addition to the first reagent. In a case where the anti-L chain λ antibody is used in combination with the anti-L chain κ antibody, the anti-L chain λ antibody and the anti-L chain κ antibody may be included in a form in which the total amount of the anti-L chain λ antibody and the anti-L chain κ antibody can be adjusted to the above-described preferred concentration. In the latex turbidimetric immunoassay method according to the present invention, the anti-L chain λ antibody is preferably an anti-L chain λ monoclonal antibody, and the anti-L chain κ antibody is also preferably an anti-L chain κ monoclonal antibody.

In the LTIA reagent according to the present invention, the concentration of the anti-L chain λ monoclonal antibody included in the general first reagent is 1 to 2,000 µg/mL, preferably 5 to 1,500 µg/mL, more preferably 10 to 1,000 µg/mL, still more preferably 15 to 800 µg/mL, and most preferably 25 to 800 µg/mL, but is not limited to this concentration.

In addition, in a case where a sample diluent liquid, a pretreatment liquid, or the like is added to a sample before the sample is subjected to the LTIA method, the anti-L chain λ antibody may be included in the pretreatment liquid, and the concentration of the anti-L chain λ antibody in the pretreatment liquid is 1 to 2,000 µg/mL, preferably 5 to 1,500 µg/mL, more preferably 10 to 1,000 µg/mL, still more preferably 15 to 800 µg/mL, and most preferably 25 to 800 µg/mL, but is not limited to this concentration.

Similarly, the concentration of the anti-L chain κ monoclonal antibody included in the general first reagent is 1 to 2,000 µg/mL, preferably 5 to 1,500 µg/mL, more preferably 10 to 1,000 µg/mL, still more preferably 15 to 800 µg/mL, and most preferably 25 to 800 µg/mL, but is not limited to this concentration.

In addition, in a case where a sample diluent liquid, a pretreatment liquid, or the like is added to a sample before the sample is subjected to the LTIA method, the anti-L chain κ antibody may be included in the pretreatment liquid, and the concentration of the anti-L chain κ antibody in the pretreatment liquid is 1 to 2,000 µg/mL, preferably 5 to 1,500 µg/mL, more preferably 10 to 1,000 µg/mL, still more preferably 15 to 800 µg/mL, and most preferably 25 to 800 µg/mL, but is not limited to this concentration.

In a case where the anti-L chain λ antibody is used in combination with the anti-L chain κ antibody, the total amount of the anti-L chain λ antibody and the anti-L chain κ antibody may be within the above-described concentration range.

As the immunoassay particles used in the present invention, known particles can be used in addition to the latex particles, as long as they can carry a binding partner specific to a target substance to be measured. For example, inorganic particles such as metal colloids, silica, and carbon can also be used as the immunoassay particles according to the present invention.

The size of the immunoassay particles can be appropriately selected from a range of 0.05 to 1 µm in consideration of the optical measurement method to be used (for example, a turbidimetric method for measuring transmitted light, a scattering method for measuring scattered light, and the like) such that a desired measurement sensitivity, a measurement range, and the like can be obtained. In the optical measurement in the automatic analysis device, an average particle diameter of 0.1 to 0.4 µm is generally used, but the present invention is not limited thereto.

### <ELISA method>

The ELISA method is a method of detecting an enzyme activity by using a combination of various antigen-antibody reactions and finally incorporating an enzyme-labeled antigen or antibody into a reaction system. In the detection of enzyme activity, a substrate whose absorption spectrum changes by a reaction is used, and examples thereof include a direct method, an indirect method, a sandwich method, and a competition method, which are based on a combination of an antigen-antibody reaction.

Examples of a reagent in a case where the immunoassay method according to the present invention is a sandwich ELISA method are described.

(a) An insoluble carrier on which an antibody that reacts with a target substance to be measured is immobilized
(b) An antibody that is labeled with a labeling substance and reacts with a target substance to be measured

As the insoluble carrier of (a), a plate is preferable, and the labeling substance can be appropriately selected and used. The antibody immobilized on the insoluble carrier captures a target substance to be measured in a solution containing a sample and forms a complex on the insoluble carrier. The antibody labeled with the labeling substance binds to the captured target substance to be measured to form a sandwich with the above-described complex. The target substance to be measured in the sample can be measured by measuring the amount of the labeling substance according to a method corresponding to the labeling substance. As a specific method such as a method of immobilizing an antibody on an insoluble carrier and a method of binding an antibody to a labeling substance, a method well known to those skilled in the art can be used without particular limitation.

In the present ELISA method, both or either of the anti-L chain λ monoclonal antibody and the anti-L chain κ monoclonal antibody according to the present invention can be present in the immunoreaction system, for example, by adding them to a sample diluent liquid, a pretreatment liquid, or the like, or adding them to a solution in which an antigen-antibody reaction is performed. In the ELISA method, the anti-L chain λ antibody is preferably an anti-L chain λ monoclonal antibody, and the anti-L chain κ antibody is preferably an anti-L chain κ monoclonal antibody.

### <Immunochromatography method>

A reagent configuration (test piece configuration) in a case where the immunoassay method according to the present invention is an immunochromatography method will be described.

### Immunochromatographic test piece;

In a case where an antibody is used as a specific binding partner, the test piece is a test piece that has, in order in a development direction of a solution containing a sample, "1. sample supply site", "2. site holding a labeled antibody (labeled antibody holding site)", and "3. site for immobilizing an antibody for capturing a complex formed by the labeled antibody and a target substance to be measured (capture antibody site)" on a sheet-shaped insoluble carrier such as a porous membrane.

In the immunochromatography method, at least test piece described above is included, in a case where a predetermined amount of a sample containing a target substance to be measured is added to a sample supply site, the sample enters a label holding site because of a capillary action, and the target substance to be measured and the labeled antibody bind to each other to form a complex. In a case where the complex is developed on the membrane and enters the capture antibody site, the complex is captured by the antibody (the capture antibody) immobilized on the membrane, and a complex of the capture antibody - the target substance to be measured - the labeled antibody is formed. Then, the target substance to be measured can be detected by detecting the label by any method (for example, in a case of a visible label such as a gold colloid, an aggregation image thereof, and in a case of an enzyme, a color forming reaction by adding a substrate).

In the present immunochromatography method, both or either of the anti-L chain λ monoclonal antibody and the anti-L chain κ monoclonal antibody according to the present invention can be present within the reaction system, for example, by adding the antibodies to a sample diluent liquid, a pretreatment liquid, or the like, or by including the antibodies in a sample supply site or a label holding site and drying and holding the antibodies. In the above-described immunochromatography method, the anti-L chain λ antibody is preferably an anti-L chain λ monoclonal antibody, and the anti-L chain κ antibody is preferably an anti-L chain κ monoclonal antibody.

### <Chemiluminescence method>

A target substance to be measured is reacted with magnetic particles to which an antigen or an antibody is bound to form a complex, and then an unreacted substance is removed by magnetism. The method is a method of adding a reagent containing a labeled antibody, removing an unreacted substance with a magnet, then adding a luminescent reagent, and measuring the amount of luminescence. A case where an enzyme is used as a label is referred to as a chemiluminescent enzyme immunoassay (CLEIA) method. In addition, a case where the luminescence intensity is measured by an electrochemical reaction using a metal complex such as a ruthenium pyridine complex as a label is referred to as an electro chemiluminescence immunoassay (ECLIA) method. In addition, a case where a chemiluminescent substance is used as a label is referred to as a chemiluminescent immunoassay (CLIA) method.

Examples of a reagent in a case where the immunoassay method according to the present invention is a CLEIA method are described.
(a) Magnetic particles on which an antibody (or an antigen) that reacts with a target substance to be measured is immobilized
(b) An antibody (or an antigen) that is enzyme-labeled and reacts with a target substance to be measured
(c) Luminescent reagent

The antibody immobilized on the magnetic particles captures a target substance to be measured in a solution containing a sample to form a complex. The antibody labeled with an enzyme-labeled substance binds to the captured target substance to be measured to form a sandwich with the above-described complex. The luminescence amount is measured by reacting the enzyme-labeled substance with a luminescent reagent, thereby the target substance to be measured in the sample can be measured.

In the present CLEIA method, both or either of the anti-L chain λ monoclonal antibody and the anti-L chain κ monoclonal antibody according to the present invention can be present in the immunoreaction system, for example, by adding them to a sample diluent liquid, a pretreatment liquid, or the like, or adding them to a solution in which an antigen-antibody reaction is performed. In the above-described chemiluminescence method, the anti-L chain λ antibody is preferably an anti-L chain λ monoclonal antibody, and the anti-L chain κ antibody is preferably an anti-L chain κ monoclonal antibody.

### <Specific binding partner>

In the present invention, examples of the specific binding partner for the target substance to be measured include proteins other than the anti-L chain λ monoclonal antibody and the anti-L chain κ monoclonal antibody, peptides, amino acids, lipids, carbohydrates, nucleic acids, and haptens. The specific binding partner is not particularly limited by the high or low molecular weight and the origin such as natural or synthetic, but examples thereof include an antibody or an antigen that can be used in an immunoassay method using an immunoreaction.

The antibody may be a polyclonal antibody or a monoclonal antibody. The monoclonal antibody is more preferable.

As the antibody according to the present invention, a functional fragment of an antibody having an antigen-antibody reaction activity can also be used in addition to the entire antibody molecule. The antibody may be an antibody (a chimeric antibody, a humanized antibody, a fully human antibody, or the like) that is changed to an amino acid sequence of an animal species different from an animal that is immunized with an immunogen (a target substance to be measured) by a gene recombination technology or the like, in addition to those obtained through an immune step on a general animal (mouse, goat, sheep, or the like). Examples of the functional fragment of an antibody include F(ab')₂, Fab', or a single-chain antibody (scFv), which is fragment having an antigen-antibody reaction activity, a variable domain of heavy chain of heavy chain antibody (VHH antibody), and a new antigen receptor (IgNAR) antibody. Functional fragments of these antibodies can be produced by treating the antibody obtained as described above with a proteolytic enzyme (for example, pepsin, papain, or the like).

### <Sample>

Examples of the sample containing the target substance to be measured according to the present invention include blood, serum, blood plasma, culture supernatant, urine, cerebrospinal fluid, saliva, sweat, ascites, and an extract liquid of cells or tissues of a human or an animal.

In addition, the sample in the present invention includes not only the sample itself obtained from a living body but also a sample subjected to pretreatment such as dilution and purification. In addition, the sample in the present invention may be any sample such as a sample immediately after collection or a sample after a certain period of time has elapsed after collection, but the present invention has a suppressing effect on non-specific factors that still exist even after a certain period of time has elapsed after collection.

In addition, the suppressing effect is also exhibited with respect to a non-specific factor present in a sample that has been frozen and stored for a certain period of time and then thawed or a sample that has been repeatedly frozen and thawed. Examples of the certain time include immediately after the collection, one day after the collection, one week after the collection, one month after the collection, and the like.

### <Target substance to be measured>

The immunoassay reagent according to the present invention can use various substances contained in the sample as a target substance to be measured. The target substance to be measured may be any substance that does not react with the anti-L chain λ antibody and the anti-L chain κ antibody used for suppressing the non-specific reaction, that is, any substance other than the immunoglobulin L chain lambda chain and the immunoglobulin L chain kappa chain. Examples thereof include proteins other than those described above, peptides, amino acids, lipids, carbohydrates, nucleic acids, and haptens, but the substance is not particularly limited as long as it is a substance that can be theoretically measured. Examples thereof include C-reactive protein (CRP), Lp(a), matrix metalloproteinase 3 (MMP3), antiphospholipid antibody, type IV collagen, PSA, brain natriuretic peptide (BNP), insulin, albumin, cystatin C, rheumatoid factor (RF), KL-6, procalcitonin, FDP, D-dimer, soluble fibrin (SF), thrombin-antithrombin III complex (TAT), PAI-1, phenytoin, phenobarbital, carbamazepine, valproic acid, theophylline, thymus and activation-regulated chemokine (TARC), soluble interleukin-2 receptor (sIL-2R), pulmonary surfactant protein D (SP-D), and the like.

Provided that an anti-immunoglobulin L chain lambda antibody and an anti-immunoglobulin L chain kappa antibody is excluded from the target substance to be measured. The anti-immunoglobulin L chain lambda antibody and the anti-immunoglobulin L chain kappa antibody described herein are not limited to monoclonal antibodies and also include polyclonal antibodies.

<Composition and non-specific reaction suppressing agent>

The present invention also provides a composition containing both or either of an anti-immunoglobulin L chain lambda monoclonal antibody or an anti-immunoglobulin L chain kappa monoclonal antibody.

The composition can be used in an immunoassay method as a non-specific reaction suppressing agent containing, for example, both or either of an anti-immunoglobulin L chain lambda monoclonal antibody or an anti-immunoglobulin L chain kappa monoclonal antibody as an effective component.

**In** the present invention, the suppression of the non-specific reaction means that the non-specific reaction suppressing agent acts on a factor (non-specific factor) that causes the above-described non-specific reaction in a biological sample, and suppresses the influence on the measurement due to a reaction other than the immunoreaction between the target substance to be measured and the specific binding partner. Therefore, in the present invention, whether or not the candidate substance as the non-specific reaction suppressing agent has an effect of suppressing a non-specific reaction can be determined by comparing a case where the candidate substance is added with a case where the candidate substance is not added, based on a measured value (hereinafter, referred to as a control method measured value) in a case where the measurement is carried out by a method in which a non-specific reaction does not occur (or a method in which a non-specific reaction is less likely to occur (in Examples, the CLEIA method)) in a certain sample, and observing whether or not the measured value approaches the control method measured value. That is, in the target measuring method, in a case where the measured value in a case where the candidate substance is added is close to the control method measured value as compared with the measured value in a case where the candidate substance is not added, it can be determined that the candidate substance has a non-specific reaction suppressing effect in the measuring method, and the candidate substance can be the non-specific reaction suppressing agent.

The composition and the non-specific reaction suppressing agent in the present invention target both a factor that causes a so-called positive measurement error, which determines a target substance to be measured to have a value higher than the original content, and a factor that causes a so-called negative measurement error, which determines a target substance to be measured to have a value lower than the original value, because of any component contained in a biological sample. Among these, the non-specific reaction suppressing agent has a particularly effect on non-specific factors that cannot be suppressed even by a commercially available non-specific reaction suppressing agent such as HBR-1. In addition, the non-specific factor suppressing agent has an effect on a non-specific factor that is included in a so-called discrepant sample and causes a positive measurement error that causes a measurement value to be abnormally high or a negative measurement error that causes a measurement value to be abnormally low.

The composition and the non-specific reaction suppressing agent according to the present invention may contain a substance capable of suppressing a reaction due to a non-specific factor derived from a sample, which is determined as described above, and contain at least an anti-L chain λ monoclonal antibody or an anti-L chain κ monoclonal antibody as an effective component. The non-specific reaction suppressing agent according to the present invention can be used as it is in the configuration including the anti-L chain λ monoclonal antibody and the anti-L chain κ monoclonal antibody, the anti-L chain λ monoclonal antibody, or the anti-L chain κ monoclonal antibody in the above-described reagent configuration.

The non-specific reaction suppressing agent according to the present invention may contain buffers, proteins, peptides, amino acids, nucleic acids, lipids, phospholipids, sugars, inorganic salts, polymer compounds, surfactants, other non-specific reaction suppressing agents (such as an anti-C3d antibody or an anti-human IgM antibody), preservatives, or the like, as long as the non-specific reaction suppressing effect is not impaired.

### <Method of suppressing non-specific reaction>

The method of suppressing a non-specific reaction according to the present invention is a method of suppressing a non-specific reaction due to a sample by performing an immunoreaction in the presence of an anti-L chain λ monoclonal antibody and an anti-L chain κ monoclonal antibody, in the presence of an anti-L chain λ monoclonal antibody, or in the presence of an anti-L chain κ monoclonal antibody.

### [Reference Documents]

1: Edward A. Greenfield (Ed.) (2012) Antibodies: A Laboratory Manual (Second Edition), Cold Spring Harbor Laboratory Press, 750pp.
2:Jerry A. Katzmann, Raynell J. Clark, Roshini S. Abraham, Sandra Bryant, James F. Lymp, Arthur R. Bradwell, and Robert A. Kyle (2002) Serum Reference Intervals and Diagnostic Ranges for Free κ and Free λ Immunoglobulin Light Chains: Relative Sensitivity for Detection of Monoclonal Light Chains, Clinical Chemistry, 48 (9): 1437-1444.

### Examples

Hereinafter, the present invention will be described in detail with reference to Examples, but the present invention is not limited to the following Examples.

### [Reference Example 1] (Production of human IgA immune-derived monoclonal antibody)

### 1. Production of human IgA immune animal-derived hybridoma

### (1) Immunization in animal

As an immunogen, commercially available human IgA (manufactured by Bethyl Laboratories, Inc.) was used. An emulsion prepared by mixing equal amounts of an immunogen diluted with a 20 mM phosphate buffer solution (pH 7.2; hereinafter, referred to as PBS) containing 150 mM sodium chloride and a complete Freund's adjuvant was used, and 30 µg per Balb/cAJcl mouse was injected. Furthermore, for the second and subsequent times, an imcomplete Freund's adjuvant was used, and injection of 20 µg per mouse was repeated 3 to 5 times at intervals of 7 to 10 days. The antibody titer in the antiserum obtained by collecting blood from the caudal vein was measured by the antigen-immobilized ELISA method described later.

### (2) Evaluation of antibody titer in immunized animal serum (antigen-immobilized ELISA method)

The existence of the antibody against human IgA in the immunized animal serum was confirmed by an ELISA method (antigen-immobilized ELISA method) in which an immunogen was immobilized. The details of the antigen-immobilized ELISA method are as follows.

### (2-1) Production of plate for antigen-immobilized ELISA

Human IgA, which is an immunogen, was dissolved in PBS to a concentration of 1.0 µg/mL, 50 µL of the obtained dissolved solution was dispensed into each well of a 96-well microplate, and the mixture was allowed to stand at room temperature for 2 hours.

Each of the wells were washed three times with 400 µL of PBS containing 0.05% Tween (registered trademark) 20 (hereinafter, referred to as PBST), and then 100 µL of PBST containing 1% bovine serum albumin (hereinafter, referred to as BSA-PBST) was added thereto, and blocking was performed at room temperature for 1 hour. This was used as a plate for ELISA.

### (2-2) Antigen-immobilized ELISA method

The BSA-PBST was removed from each well of the ELISA plate, then 50 µL of an immune animal antiserum and a non-immune animal serum, which had been subjected to stepwise dilution with BSA-PBST, were added to each well, and the mixture was allowed to stand at room temperature for 1 hour. After washing each well with 400 µL of PBST three times, 50 µL of HRP-labeled anti-mouse IgG (H & L) (manufactured by Southern Biotechnology Associates Inc.) diluted 10,000 times with BSA-PBST was dispensed into each well, and the mixture was allowed to stand at room temperature for 1 hour. The wells were washed three times with 400 µL of PBST, 50 µL of a citrate buffer solution (pH 5.0) containing 0.2% ortho-phenylenediamine and 0.02% hydrogen peroxide was added thereto, the mixture was allowed to stand at room temperature for 10 minutes, 50 µL of 1.5 N sulfuric acid was added thereto to stop the enzymatic reaction, and the absorbance at a wavelength of 492 nm was measured. As a result of the measurement, the spleen and lymph nodes were excised from the mice in which an increase in antibody titer was confirmed, and spleen- and lymph node-derived cells were prepared and used for cell fusion.

### (3) Cell fusion

The spleen-derived cells or lymph node-derived cells and the myeloma cells were mixed at a ratio of 6:1 in terms of the number of cells, and cell fusion was performed by the PEG method. The cells obtained by cell fusion were suspended in a HAT medium and cultured in a CO₂ incubator at 37°C and 5% CO₂ for 8 days to obtain a fusion cell (hybridoma).

### 2. Sorting of hybridoma (antigen solid-phase ELISA method)

The existence of the antibody against IgA in the culture supernatant of the hybridoma was confirmed by an ELISA method (antigen-immobilized ELISA method) in which an immunogen was immobilized. The details of the antigen-immobilized ELISA method are as follows.

### (1) Production of plate for antigen-immobilized ELISA

IgA, which is an immunogen, was dissolved in PBS to a concentration of 1.0 µg/mL, 50 µL of the obtained dissolved solution was dispensed into each well of a 96-well microplate, and the mixture was allowed to stand at room temperature for 2 hours.

Each of the wells was washed three times with 400 µL of PBST, 100 µL of BSA-PBST was then added thereto, and blocking was performed at room temperature for 1 hour. This was used as a plate for ELISA.

### (2) Antigen-immobilized ELISA method

The BSA-PBST was removed from each well of the ELISA plate, then 50 µL of culture supernatants of the hybridoma, which had been subjected to stepwise dilution with BSA-PBST, were added to each well, and the mixture was allowed to stand at room temperature for 1 hour. Each of the wells was washed three times with 400 µL of PBST, 50 µL of HRP-labeled anti-mouse IgG (H & L) diluted 10,000 times with BSA-PBST was dispensed into each well, and the mixture was allowed to stand at room temperature for 1 hour. The wells were washed three times with 400 µL of PBST, 50 µL of a citrate buffer solution (pH 5.0) containing 0.2% ortho-phenylenediamine and 0.02% hydrogen peroxide was added thereto, the mixture was allowed to stand at room temperature for 10 minutes, 50 µL of 1.5 N sulfuric acid was added thereto to stop the enzymatic reaction, and the absorbance at a wavelength of 492 nm was measured.

As a result of the measurement, a well having a high absorbance was selected as a well (positive well) in which the human IgA antibody-producing hybridoma was present.

### 3. Cloning

Using the above-described anti-human IgA antibody-producing hybridoma selected in 2., the monocloning of the hybridoma and the purification of the monoclonal antibody were performed. The cloning was performed by a conventional method (a limiting dilution method), and positive wells were sorted by the same method as the above-described antigen solid-phase ELISA method, and finally, a plurality of monoclonal antibody-producing hybridomas were obtained. The monoclonal antibody produced by the obtained hybridoma S062XX (XX is a number) was referred to as an S062XX antibody.

### [Reference Example 2] (Evaluation of specificity of human IgA immune-derived monoclonal antibody)

### 1. Evaluation of specificity of human IgA immune-derived monoclonal antibody (antigen solid-phase ELISA method)

The specificity of the human IgA immune-derived monoclonal antibody was confirmed by an ELISA method (antigen-immobilized ELISA method) in which an antigen was immobilized. The details of the antigen-immobilized ELISA method are as follows.

### (1) Production of a plate for antigen-immobilized ELISA method

Antigens (IgG Kappa and IgG Lambda (both manufactured by Bethyl Laboratories Inc.), and IgA Kappa and IgA Lambda (both manufactured by Southern Biotechnology Associates Inc.)) for evaluating a specificity were dissolved in PBS to a concentration of 1.0 µg/mL, 50 µL of the obtained dissolved solution was dispensed into each well of a 96-well microplate, and the mixture was allowed to stand at room temperature for 2 hours.

Each of the wells was washed three times with 400 µL of PBST, 100 µL of BSA-PBST was then added thereto, and blocking was performed at room temperature for 1 hour. This was used as a plate for ELISA.

### (2) Antigen-immobilized ELISA method

The BSA-PBST was removed from each well of the ELISA plate, then 50 µL of antibodies diluted to 1.0 µg/mL and 0.2 µg/mL with BSA-PBST were added to each well, and the mixture was allowed to stand at room temperature for 1 hour. Each of the wells was washed three times with 400 µL of PBST, 50 µL of HRP-labeled anti-mouse IgG (H & L) diluted 9,500 times with BSA-PBST was dispensed into each well, and the mixture was allowed to stand at room temperature for 1 hour. The wells were washed three times with 400 µL of PBST, 50 µL of a citrate buffer solution (pH 5.0) containing 0.2% ortho-phenylenediamine and 0.02% hydrogen peroxide was added thereto, the mixture was allowed to stand at room temperature for 10 minutes, 50 µL of 1.5 N sulfuric acid was added thereto to stop the enzymatic reaction, and the absorbance at a wavelength of 492 nm was measured. The results of the measurement are shown in Table 1.

### 2. Specificity evaluation result

Among the plurality of human IgA immune-derived monoclonal antibodies, an antibody (S06201, S06216) specific to IgA, which reacts with IgA Kappa and IgA Lambda and does not react with IgG Kappa and IgG Lambda, an antibody (S06205, S06204) specific to L chain κ, which reacts with IgG Kappa and IgA Kappa and does not react with IgG Lambda and IgA Lambda, and an antibody (S06215, S06208) specific to L chain λ, which reacts with IgG Lambda and IgA Lambda and does not react with IgG Kappa and IgA Kappa, were recognized. The reason why it is determined that the antibody is an antibody specific to IgA is considered as follows. That is, the fact that both antigens of the κ chain and the λ chain of IgA react is considered to be an antibody that reacts with the H chain, not an antibody that reacts with the L chain. In addition, since the antibody did not react with the λ chain and the κ chain of IgG, it was considered to be an antibody specific to IgA.

In this way, the anti-L chain λ monoclonal antibody and the anti-L chain κ monoclonal antibody according to the present invention were sorted from the human IgA immune-derived monoclonal antibodies, and the non-specific reaction suppressing effect in the immunoassay method was evaluated.

**[Table 1]**

| Evaluation of specificity of human IgA immune-derived monoclonal antibody | | | | | |
|---|---|---|---|---|---|
| Antibody name | Antibody concentration | Immobilized antibody | | | |
| | | IgG, kappa | IgG, lambda | IgA, kappa | IgA, lambda |
| S06201 | 1.0 µg/mL | 0.043 | 0.043 | 1.359 | 1.384 |
| | 0.2 µg/mL | 0.042 | 0.041 | 1.140 | 1.145 |
| S06216 | 1.0 µg/mL | 0.042 | 0.042 | 1.061 | 1.237 |
| | 0.2 µg/mL | 0.041 | 0.041 | 0.755 | 0.869 |
| S06205 | 1.0 µg/mL | 1.410 | 0.043 | 1.301 | 0.072 |
| | 0.2 µg/mL | 0.814 | 0.043 | 0.877 | 0.062 |
| S06204 | 1.0 µg/mL | 1.014 | 0.052 | 1.003 | 0.051 |
| | 0.2 µg/mL | 0.562 | 0.044 | 0.598 | 0.051 |
| S06215 | 1.0 µg/mL | 0.041 | 1.587 | 0.051 | 1.563 |
| | 0.2 µg/mL | 0.040 | 1.078 | 0.047 | 1.128 |
| S06208 | 1.0 µg/mL | 0.103 | 1.178 | 0.072 | 1.072 |
| | 0.2 µg/mL | 0.056 | 1.172 | 0.049 | 1.070 |

| | | | | | |
|---|---|---|---|---|---|
| Abs. (OD) at 492 nm | | | | | |

### [Experimental Example 1: suppression of non-specific reaction in LTIA method: measurement of sIL-2R]

The test in which the sIL-2R concentration in a sample is measured using a reagent according to the LTIA method to which the anti-L chain λ monoclonal antibody, the anti-L chain κ monoclonal antibody, and other non-specific reaction suppressing agents according to the present invention are added is shown. As a sample, a sample (control sample: samples 1 to 6) showing a value close to the measured value by the chemiluminescent enzyme immunoassay method (CLEIA method) (Control Example 1) and a sample (discrepant sample: samples 7 to 11) exhibiting a non-specific reaction and significantly deviating from the measured value of Control Example 1 were used.

### [Control Example 1] (Measurement by CLEIA method)

### 1. Measuring method

### 1-1. Measurement reagent Lumipulse presto (registered trademark) IL-2R (Fujirebio Inc.)

### 1-2. Sample

Samples (serum) 1 to 11

### 1-3. Measurement procedure

The measurement was performed according to the package insert of the measurement reagent using Lumipulse (registered trademark)-L2400 (Fujirebio Inc.).

### 2. Measurement results

Measurement results were shown in Table 2.

In the CLEIA method shown in Control Example 1, a B/F separation operation is performed and a washing step is included. Therefore, since this method was a measuring method that is less susceptible to the influence of a non-specific reaction derived from a sample, it was used as a control example.

### [Comparative Example 1] (Measurement by LTIA method: no addition of non-specific reaction suppressing agent)

### 1. Measuring method

### 1-1. Measurement Reagent

The following first reagent (buffer solution) and second reagent (anti sIL-2R antibody-sensitized latex particle solution) were prepared according to the method described in JP2017-181377A.

### 1-2. Sample

The same as the sample of Control Example 1.

### 1-3. Measurement procedure

The first reagent and the second reagent were combined, and the sIL-2R concentration in the sample was measured using a Hitachi 7180 automatic analyzer. Specifically, 120 µL of the first reagent was added to 5.6 µL of the sample, the mixture was kept at 37°C for 5 minutes, 40 µL of the second reagent was added thereto, and the mixture was stirred. The absorbance change associated with the aggregation formation was measured at a main wavelength of 570 nm and a sub wavelength of 800 nm for 5 minutes thereafter, and the amount of the absorbance change was applied to a calibration curve obtained by measuring standard substances with a known concentration to calculate a measured value.

### 2. Measurement results

Measurement results were shown in Table 2.

### [Comparative Example 2] (Measurement by LTIA method: addition of HBR-1)

The measurement was performed by the same method as in Comparative Example 1, except that HBR-1 (manufactured by SCANTIBODIES LABORATORY, INC.), a commercially available non-specific reaction suppressing agent, was added to the first reagent shown in Comparative Example 1 such that the concentration of the HBR-1 was 200 µg/mL. Measurement results were shown in Table 2.

### [Comparative Example 3] (Measurement by LTIA method: addition of anti-human IgG, IgA, and IgM polyclonal antibodies)

The measurement was performed by the same method as in Comparative Example 1, except that an anti-human IgG polyclonal antibody, an anti-human IgA polyclonal antibody, and an anti-human IgM polyclonal antibody (manufactured by SeraCare Life Sciences, Inc. S), which was commercially available, were added to the first reagent shown in Comparative Example 1 such that the concentration of each antibody was 200 µg/mL. Measurement results were shown in Table 2.

### [Example 1] (Measurement by LTIA method: addition of anti-L chain λ monoclonal antibody)

The measurement was performed by the same method as in Comparative Example 1, except that the anti-L chain λ monoclonal antibody (S06215 antibody prepared in Reference Example 1) was added to the first reagent shown in Comparative Example 1 such that the concentration of the antibody was 200 µg/mL. Measurement results were shown in Table 2.

### [Example 2] (Measurement by LTIA method: addition of anti-L chain κ monoclonal antibody)

The measurement was performed by the same method as in Comparative Example 1, except that the anti-L chain κ monoclonal antibody (S06205 antibody prepared in Reference Example 1) was added to the first reagent shown in Comparative Example 1 such that the concentration of the antibody was 200 µg/mL. Measurement results were shown in Table 2.

### [Example 3] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 1 such that the concentration of each antibody was 200 µg/mL. Measurement results were shown in Table 2.

**[Table 2]**

| Measurement results of sIL-2R (U/mL) | | | | | | |
|---|---|---|---|---|---|---|
| | | | Comparative Example 1 | Comparative Example 2 | Comparative Example 3 | Control Example 1 |
| Measuring method | | | LTIA method | LTIA method | LTIA method | CLEIA method |
| Additive and addition concentration | | | No additive | HBR-1 200 µg/mL | Anti-human IgG, IgA, or IgM polyclonal antibody 200 µg/mL | - |
| Control sample | Sample number | 1 | 288 | 287 | 369 | 296 |
| | | 2 | 600 | 619 | 736 | 598 |
| | | 3 | 438 | 471 | 558 | 438 |
| | | 4 | 708 | 736 | 881 | 670 |
| | | 5 | 915 | 934 | 1113 | 906 |
| | | 6 | 2038 | 2047 | 2735 | 2140 |
| Discrepant sample | Sample number | 7 | 1814 | 2037 | 1060 | 672 |
| | | 8 | 748 | 760 | 669 | 601 |
| | | 9 | 512 | 551 | 558 | 432 |
| | | 10 | 1857 | 1883 | 2385 | 1640 |
| | | 11 | 1227 | 1264 | 1455 | 1070 |
| | | | Example 1 | Example 2 | Example 3 | |
| Measuring method | | | LTIA method | LTIA method | LTIA method | |
| Additive and addition concentration | | | Anti-L chain λ monoclonal antibody 200 µg/mL addition | Anti-L chain κ monoclonal antibody 200 µg/mL addition | Anti-L chain λ monoclonal antibody 200 µg/mL | |
| | | | | | Anti-L chain κ monoclonal antibody 200 µg/mL | |
| Control sample | Sample number | 1 | 273 | 290 | 290 | |
| | | 2 | 588 | 615 | 622 | |
| | | 3 | 440 | 444 | 460 | |
| | | 4 | 710 | 722 | 734 | |
| | | 5 | 922 | 942 | 925 | |
| | | 6 | 2037 | 2096 | 2142 | |
| Discrepant sample | Sample number | 7 | 587 | 1809 | 617 | |
| | | 8 | 607 | 751 | 667 | |
| | | 9 | 390 | 521 | 420 | |
| | | 10 | 1929 | 1529 | 1567 | |
| | | 11 | 1245 | 1068 | 1119 | |

### <Results and Considerations>

The effects of the present invention were considered from the results of Control Example 1, Comparative Examples 1 to 3, and Examples 1 to 3.

(1) The measurement results of the control samples (sample numbers 1 to 6) were verified.
   In the control sample, the measured value of the CLEIA method (Control Example 1) and the measured value of the LTIA method to which the non-specific reaction suppressing agent was not added (Comparative Example 1) were generally equivalent. In addition, the measured values of the LTIA method to which the anti-L chain λ monoclonal antibody according to the present invention was added (Example 1), the measured values of the LTIA method to which the anti-L chain κ monoclonal antibody was added (Example 2), and the measured values of the LTIA method to which the anti-L chain λ monoclonal antibody and the anti-L chain κ monoclonal antibody were used in combination (Example 3) were also generally equivalent to those of Control Example 1 and Comparative Example 1. Therefore, it was found that the addition of the anti-L chain λ monoclonal antibody or the anti-L chain κ monoclonal antibody does not affect the measured value of the sample that does not exhibit a non-specific reaction. On the other hand, in the measured value of the LTIA method to which the anti-human IgG polyclonal antibody, the anti-human IgA polyclonal antibody, and the anti-human IgM polyclonal antibody were added (Comparative Example 3), a tendency of further deviation was observed as compared with that of Comparative Example 1.
(2) The measurement results of the discrepant samples (sample numbers 7 to 11) were verified.

In the sample number 7, the measured value of the CLEIA method (Control Example 1) was 672 U/mL, whereas the measured value of the LTIA method to which the non-specific reaction suppressing agent was not added (Comparative Example 1) was 1814 U/mL, and the measured values were significantly deviated. Furthermore, the measured value of the LTIA method to which HBR-1 was added (Comparative Example 2) was 2037 U/mL, and the measured values were significantly deviated from that of Control Example 1. The measured value of the LTIA method to which the anti-human IgG polyclonal antibody, the anti-human IgA polyclonal antibody, and the anti-human IgM polyclonal antibody were added (Comparative Example 3) was 1060 U/mL, and although a non-specific reaction suppressing effect was observed, the non-specific reaction suppressing effect of the present substance was insufficient because the measured value was deviated from that of Control Example 1.

On the other hand, the measured value of the LTIA method to which the anti-L chain λ monoclonal antibody according to the present invention was added (Example 1) was 587 U/mL, and the measured value of the LTIA method in which the anti-L chain λ monoclonal antibody and the anti-L chain κ monoclonal antibody were used in combination (Example 3) was 617 U/mL, which showed a tendency to approach that of Control Example 1. The same results were obtained in the samples 8 and 9.

Furthermore, in the sample number 10, the measured value of the CLEIA method (Control Example 1) was 1640 U/mL, whereas the measured value of the LTIA method to which the non-specific reaction suppressing agent was not added (Comparative Example 1) was 1857 U/mL, and the measured values were deviated. The measured value of the LTIA method to which HBR-1 was added (Comparative Example 2) was 1883 U/mL, and the measured values were significantly deviated from that of Control Example 1. The measured value of the LTIA method to which the anti-human IgG polyclonal antibody, the anti-human IgA polyclonal antibody, and the anti-human IgM polyclonal antibody were added (Comparative Example 3) was 2385 U/mL, and the degree of deviation from that of Control Example 1 increased.

On the other hand, the measured value of the LTIA method to which the anti-L chain κ monoclonal antibody according to the present invention was added (Example 2) was 1529 U/mL, and the measured value of the LTIA method in which the anti-L chain λ monoclonal antibody and the anti-L chain κ monoclonal antibody were used in combination (Example 3) was 1567 U/mL, which showed a tendency to approach that of Control Example 1. The same results were obtained in the sample 11.

(3) As described above, in the immunoassay method, by allowing the anti-L chain λ monoclonal antibody or the anti-L chain κ monoclonal antibody according to the present invention to be present within the immunoreaction system, it is possible to suppress the non-specific reaction derived from the sample.

Since the non-specific reaction that could not be suppressed even by HBR-1, an anti-human IgG polyclonal antibody, an anti-human IgA polyclonal antibody, and an anti-human IgM polyclonal antibody, which are commercially available non-specific reaction suppressing agents, could be suppressed by the present invention, the non-specific reaction suppressing effect of the anti-L chain λ monoclonal antibody or the anti-L chain κ monoclonal antibody according to the present invention was an unexpected result. In addition, by using the anti-L chain λ monoclonal antibody and the anti-L chain κ monoclonal antibody in combination, it is possible to suppress various non-specific reactions derived from the sample.

In addition, in sample numbers 7 to 9, the anti-L chain λ monoclonal antibody alone also had a non-specific reaction suppressing effect. In the sample numbers 10 and 11, the non-specific reaction suppressing effect was also exhibited by the anti-L chain κ monoclonal antibody alone. As described above, the effect of suppressing a non-specific reaction with respect to a specific sample was exhibited by the anti-L chain λ monoclonal antibody alone or the anti-L chain κ monoclonal antibody alone. On the other hand, since the effect varies depending on the sample, it is preferable to use the anti-L chain λ monoclonal antibody and the anti-L chain κ monoclonal antibody in combination.

### [Experimental Example 2: suppression of non-specific reaction in LTIA method: measurement of SP-D]

The test in which the SP-D concentration in a sample is measured using a reagent according to the LTIA method to which the anti-L chain λ monoclonal antibody, the anti-L chain κ monoclonal antibody, and other non-specific reaction suppressing agents according to the present invention are added is shown. As a sample, a sample (control sample: samples 12 to 15) showing a value close to the measured value by the chemiluminescent enzyme immunoassay method (CLEIA method) (Control Example 2) and a sample (discrepant sample: samples 16 and 17) exhibiting a non-specific reaction and significantly deviating from the measured value of Control Example 2 were used.

### [Control Example 2] (Measurement by CLEIA method)

### 1. Measuring method

### 1-1. Measurement Reagent

CL SP-D "YAMASA" NX (YAMASA SHOYU CO., LTD.)

### 1-2. Sample

Samples (serum) 12 to 17

### 1-3. Measurement procedure

The measurement was performed according to the package insert of the measurement reagent using CL-JACK NX (Minaris Medical Co., Ltd.).

### 2. Measurement results

Measurement results were shown in Table 3.

In the CLEIA method shown in Control Example 2, a B/F separation operation is performed and a washing step is included. Therefore, since this method was a measuring method that is less susceptible to the influence of a non-specific reaction derived from a sample, it was used as a control example.

### [Comparative Example 4] (Measurement by LTIA method: without addition of non-specific reaction suppressing agent)

### 1. Measuring method

### 1-1. Measurement Reagent

The following first reagent and second reagent were prepared.

### (1) First reagent

100 mM MES-NaOH (pH 6.0)
500 mM NaCl
0.5% BSA
Sensitizer

### (2) Second reagent

Anti-human SP-D monoclonal antibody-sensitized latex
5 mM MOPS-NaOH (pH 7.0)

### 1-2. Sample

The same as the sample of Control Example 2.

### 1-3. Measurement procedure

The first reagent and the second reagent were combined, and the SP-D concentration in the sample was measured using a Hitachi 7180 automatic analyzer. Specifically, 120 µL of the first reagent was added to 5.0 µL of the sample, the mixture was kept at 37°C for 5 minutes, 40 µL of the second reagent was added thereto, and the mixture was stirred. The absorbance change associated with the aggregation formation was measured at a main wavelength of 570 nm and a sub wavelength of 800 nm for 5 minutes thereafter, and the amount of the absorbance change was applied to a calibration curve obtained by measuring standard substances with a known concentration to calculate a measured value.

### 2. Measurement results

Measurement results were shown in Table 3.

### [Comparative Example 5] (Measurement by LTIA method: addition of HBR-1)

The measurement was performed by the same method as in Comparative Example 4, except that HBR-1 (SCANTIBODIES LABORATORY, INC.), a commercially available non-specific reaction suppressing agent, was added to the first reagent shown in Comparative Example 4 such that the concentration of the HBR-1 was 200 µg/mL. Measurement results were shown in Table 3.

### [Example 4] (Measurement by LTIA method: addition of anti-L chain λ monoclonal antibody)

The measurement was performed by the same method as in Comparative Example 4, except that the anti-L chain λ monoclonal antibody (S06215 antibody prepared in Reference Example 1) was added to the first reagent shown in Comparative Example 4 such that the concentration of the antibody was 200 µg/mL. Measurement results were shown in Table 3.

### [Example 5] (Measurement by LTIA method: addition of anti-L chain κ monoclonal antibody)

The measurement was performed by the same method as in Comparative Example 4, except that the anti-L chain κ monoclonal antibody (S06205 antibody prepared in Reference Example 1) was added to the first reagent shown in Comparative Example 4 such that the concentration of the antibody was 200 µg/mL. Measurement results were shown in Table 3.

### [Example 6] (Measurement by LTIA method: addition of anti-L chain λ monoclonal antibody and anti-L chain κ monoclonal antibody)

The measurement was performed by the same method as in Comparative Example 4, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 4 such that the concentration of each antibody was 200 µg/mL. Measurement results were shown in Table 3.

**[Table 3]**

| Measurement results of SP-D (ng/mL) | | | | | |
|---|---|---|---|---|---|
| | | | Comparative Example 5 | Comparative Example 6 | Control Example 2 |
| Measuring method | | | LTIA method | LTIA method | CLEIA method |
| Additive and addition concentration | | | No additive | HBR-1 200 µg/mL | - |
| Control sample | Sample number | 12 | 234.9 | 239.4 | 254.0 |
| | | 13 | 159.3 | 161.9 | 167.0 |
| | | 14 | 64.5 | 62.3 | 57.1 |
| | | 15 | 88.6 | 89.7 | 86.0 |
| Discrepant sample | Sample number | 16 | 29.0 | 25.3 | 15.0 |
| | | 17 | 37.9 | 17.2 | 15.0 |
| | | | Example 4 | Example 5 | Example 6 |
| Measuring method | | | LTIA method | LTIA method | LTIA method |
| Additive and addition concentration | | | Anti-L chain λ monoclonal antibody | Anti-L chain κ monoclonal antibody | Anti-L chain λ monoclonal antibody |
| | | | 200 µg/mL | 200 µg/mL | 200 µg/mL |
| | | | | | Anti-L chain κ monoclonal antibody 200 µg/mL |
| Control sample | Sample number | 12 | 235.3 | 236.0 | 237.6 |
| | | 13 | 154.2 | 158.4 | 158.1 |
| | | 14 | 64.1 | 63.2 | 63.8 |
| | | 15 | 88.5 | 88.4 | 90.0 |
| Discrepant sample | Sample number | 16 | 13.6 | 23.7 | 14.1 |
| | | 17 | 27.2 | 27.8 | 22.0 |

### <Results and Considerations>

The effects of the present invention were considered from the results of Control Example 2, Comparative Examples 4 and 5, and Examples 4 to 6.

(1) The measurement results of the control samples (sample numbers 12 to 15) were verified.
   In the control sample, the measured value of the CLEIA method (Control Example 2) and the measured value of the LTIA method to which the non-specific reaction suppressing agent was not added (Comparative Example 4) were generally equivalent. In addition, the measured values of the LTIA method to which the anti-L chain λ monoclonal antibody according to the present invention was added (Example 4), the measured values of the LTIA method to which the anti-L chain κ monoclonal antibody was added (Example 5), and the measured values of the LTIA method to which the anti-L chain λ monoclonal antibody and the anti-L chain κ monoclonal antibody were used in combination (Example 6) were also generally equivalent to those of Control Example 2 and Comparative Example 4. Therefore, it was found that the addition of the anti-L chain λ monoclonal antibody or the anti-L chain κ monoclonal antibody does not affect the measured value of the sample that does not exhibit a non-specific reaction.
(2) The measurement results of the discrepant samples (sample numbers 16 and 17) were verified.

In the sample number 16, the measured value of the CLEIA method (Control Example 2) was 15.0 ng/mL, whereas the measured value of the LTIA method to which the non-specific reaction suppressing agent was not added (Comparative Example 4) was 29.0 ng/mL, and the measured values were significantly deviated. Furthermore, the measured value of the LTIA method to which HBR-1 was added (Comparative Example 5) was 25.3 ng/mL, and the measured values were significantly deviated from that of Control Example 2.

On the other hand, the measured value of the LTIA method to which the anti-L chain λ monoclonal antibody according to the present invention was added (Example 4) was 13.6 ng/mL, and the measured value of the LTIA method in which the anti-L chain λ monoclonal antibody and the anti-L chain κ monoclonal antibody were used in combination (Example 6) was 14.1 ng/mL, which showed a tendency to approach that of Comparative Example 5.

Furthermore, in the sample number 17, the measured value of the CLEIA method (Control Example 2) was 15.0 ng/mL, whereas the measured value of the LTIA method to which the non-specific reaction suppressing agent was not added (Comparative Example 4) was 37.9 ng/mL, and the measured values were significantly deviated. The measured value of the LTIA method to which HBR-1 was added (Comparative Example 5) was 17.2 ng/mL, which showed a tendency to approach that of Comparative Example 5. The measured value of the LTIA method to which the anti-L chain λ monoclonal antibody according to the present invention was added (Example 4) was 27.2 ng/mL, and the measured value of the LTIA method to which the anti-L chain κ monoclonal antibody was added (Example 5) was 27.8 ng/mL, and the degree of deviation from Comparative Example 4 was reduced. Furthermore, the measured value of the LTIA method in which the anti-L chain λ monoclonal antibody and the anti-L chain κ monoclonal antibody were used in combination (Example 6) was 22.0 ng/mL, which showed a tendency to approach that of Comparative Example 4 as compared with the measured values of Examples 4 and 5.

(3) As described above, in the immunoassay method, by allowing the anti-L chain λ monoclonal antibody or the anti-L chain κ monoclonal antibody according to the present invention to be present within the immunoreaction system, it was possible to suppress the non-specific reaction derived from the sample. The non-specific reaction that could not be suppressed even by HBR-1, which is a commercially available non-specific reaction suppressing agent, could be suppressed by the present invention, the non-specific suppressing effect by the anti-L chain λ monoclonal antibody or the anti-L chain κ monoclonal antibody according to the present invention was an unexpected result. Furthermore, an additive effect of suppressing a non-specific reaction was observed by using the anti-L chain λ monoclonal antibody and the anti-L chain κ monoclonal antibody in combination.

### [Experimental Example 3: suppression of non-specific reaction in LTIA method: measurement of TARC]

The test in which the TARC concentration in a sample is measured using a reagent according to the LTIA method to which the anti-L chain λ monoclonal antibody, the anti-L chain κ monoclonal antibody, and other non-specific reaction suppressing agents according to the present invention are added is shown. As a sample, a sample (control sample: samples 18 to 23) showing a value close to the measured value by the chemiluminescent enzyme immunoassay method (CLEIA method) (Control Example 3) and a sample (discrepant sample: samples 24 to 30) exhibiting a non-specific reaction and significantly deviating from the measured value of Control Example 3 were used.

### [Control Example 3] (Measurement by CLEIA method)

### 1. Measuring method

### 1-1. Measurement reagent HISCL (registered trademark) TARC (Sysmex Corporation)

### 1-2. Sample

Samples (serum) 18 to 30

### 1-3. Measurement procedure

The measurement was performed according to the package insert of the reagent using HISCL (registered trademark)-5000 (Sysmex Corporation).

### 2. Measurement results

Measurement results were shown in Table 4.

In the CLEIA method shown in Control Example 3, a B/F separation operation is performed and a washing step is included. Therefore, since this method was a measuring method that is less susceptible to the influence of a non-specific reaction derived from a sample, it was used as a control example.

### [Comparative Example 6] (Measurement by LTIA method)

### 1. Measuring method

### 1-1. Measurement reagent

According to the method described in WO2022/009974A, an LTIA measurement reagent consisting of a first reagent (buffer solution) and a second reagent (anti-human TARC monoclonal antibody-sensitized latex solution) was prepared. In addition, HeteroBlock (manufactured by OMEGA Biologicals, Inc.), which is a commercially available non-specific reaction suppressing agent, was added to the first reagent such that the concentration of HeteroBlock was 100 µg/mL.

### 1-2. Sample

The same as the sample of Control Example 3.

### 1-3. Measurement procedure

The first reagent and the second reagent were combined, and the TARC concentration in the sample was measured using an automatic analyzer 3500. Specifically, 120 µL of the first reagent was added to 2.4 µL of the sample, the mixture was kept at 37°C for 5 minutes, 40 µL of the second reagent was added thereto, and the mixture was stirred. The absorbance change associated with the aggregation formation was measured at a main wavelength of 570 nm and a sub wavelength of 800 nm for 5 minutes thereafter, and the amount of the absorbance change was applied to a calibration curve obtained by measuring standard substances with a known concentration to calculate a measured value.

### 2. Measurement results

Measurement results were shown in Table 4.

### [Example 7] (Measurement by LTIA method: addition of anti-L chain λ monoclonal antibody)

The measurement was performed by the same method as in Comparative Example 6, except that the anti-L chain λ monoclonal antibody (S06215 antibody prepared in Reference Example 1) was added to the first reagent shown in Comparative Example 6 such that the concentration of the antibody was 50 µg/mL. Measurement results were shown in Table 4.

### [Example 8] (Measurement by LTIA method: addition of anti-L chain λ monoclonal antibody or anti-L chain κ monoclonal antibody)

The measurement was performed by the same method as in Comparative Example 6, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 6 such that the concentrations of the antibodies were 50 µg/mL. Measurement results were shown in Table 4.

**[Table 4]**

| Measurement results of TARC (pg/mL) | | | | | | |
|---|---|---|---|---|---|---|
| | | | Example 7 | Example 8 | Control Example 3 | Comparative Example 6 |
| Measuring method | | | LTIA method | LTIA method | CLEIA method | LTIA method |
| Additive and concentration | | | HeteroBlock 100 µg/mL | HeteroBlock 100 µg/mL | - | HeteroBlock 100 µg/mL |
| | | | | Anti-L chain λ monoclonal antibody 50 µg/mL | | |
| | | | Anti-L chain λ monoclonal antibody 50 µg/mL | | | |
| | | | | Anti-L chain κ monoclonal antibody 50 µg/mL | | |
| Control sample | Sample number | 18 | 358 | 289 | 316 | 266 |
| | | 19 | 646 | 633 | 536 | 662 |
| | | 20 | 510 | 469 | 538 | 466 |
| | | 21 | 294 | 221 | 275 | 266 |
| | | 22 | 531 | 436 | 492 | 534 |
| | | 23 | 1341 | 1228 | 1230 | 1317 |
| Discrepant sample | Sample number | 24 | 444 | 428 | 390 | 737 |
| | | 25 | 834 | 665 | 669 | 816 |
| | | 26 | 628 | 337 | 225 | 612 |
| | | 27 | 1045 | 423 | 410 | 1901 |
| | | 28 | 667 | 426 | 307 | 1054 |
| | | 29 | 3153 | 1044 | 751 | 7109 |
| | | 30 | 6368 | 1675 | 1466 | 8382 |

### <Results and Considerations>

The effects of the present invention were considered from the results of Control Example 3, Comparative Example 6, and Examples 7 and 8.

(1) The measurement results of the control samples (sample numbers 18 to 23) were verified.
   In the control sample, the measured value of the CLEIA method (Control Example 3) and the measured value of the LTIA method to which HeteroBlock, which is a commercially available non-specific reaction suppressing agent, was added (Comparative Example 6) were generally equivalent. In addition, the measured values of the LTIA method to which the anti-L chain λ monoclonal antibody according to the present invention was added (Example 7), and the measured values of the LTIA method to which the anti-L chain λ monoclonal antibody and the anti-L chain κ monoclonal antibody were used in combination (Example 8) were also generally equivalent to those of Control Example 3 and Comparative Example 6. Therefore, it was found that the addition of the anti-L chain λ monoclonal antibody or the anti-L chain κ monoclonal antibody does not affect the measured value of the sample that does not exhibit a non-specific reaction.
(2) The measurement results of the discrepant samples (sample numbers 24 to 30) were verified.

In the sample number 24, the measured value of the CLEIA method (Control Example 3) was 390 pg/mL, whereas the measured value of the LTIA method to which HeteroBlock was added (Comparative Example 6) was 737 pg/mL, and the measured values were significantly deviated.

On the other hand, the measured value of the LTIA method to which the anti-L chain λ monoclonal antibody according to the present invention was added (Example 7) was 444 pg/mL, and the measured value of the LTIA method in which the anti-L chain λ monoclonal antibody and the anti-L chain κ monoclonal antibody were used in combination (Example 8) was 428 pg/mL, which showed a tendency to approach that of Control Example 3.

Furthermore, in the sample number 25, the measured value of the CLEIA method (Control Example 3) was 669 pg/mL, whereas the measured value of the LTIA method to which HeteroBlock was added (Comparative Example 6) was 816 pg/mL, and the measured values were significantly deviated. The measured value of the LTIA method to which the anti-L chain λ monoclonal antibody according to the present invention was added (Example 7) was 834 pg/mL and the measured value was not changed, and the measured value of the LTIA method in which the anti-L chain λ monoclonal antibody and the anti-L chain κ monoclonal antibody were used in combination (Example 8) was 665 pg/mL, which showed a tendency to approach that of Control Example 3. The same results were obtained in the sample number 26.

In the sample number 27, the measured value of the CLEIA method (Control Example 3) was 410 pg/mL, whereas the measured value of the LTIA method to which HeteroBlock was added (Comparative Example 6) was 1,901 pg/mL, and the measured values were significantly deviated. The measured value of the LTIA method to which the anti-L chain λ monoclonal antibody according to the present invention was added (Example 7) was 1,045 pg/mL and the degree of deviation was reduced, and the measured value of the LTIA method in which the anti-L chain λ monoclonal antibody and the anti-L chain κ monoclonal antibody were used in combination (Example 8) was 423 pg/mL and the degree of deviation was reduced, which was almost the same as that of Control Example 3. The same tendency was obtained in the samples 28 to 30.

(3) As described above, in the immunoassay method, by allowing the anti-L chain λ monoclonal antibody or the anti-L chain κ monoclonal antibody according to the present invention to be present within the immunoreaction system, it was possible to suppress the non-specific reaction derived from the sample. The non-specific reaction that could not be suppressed even by HeteroBlock, which is a commercially available non-specific reaction suppressing agent, could be suppressed by the present invention, the non-specific reaction suppressing effect by the anti-L chain λ monoclonal antibody or the anti-L chain κ monoclonal antibody according to the present invention was an unexpected result. Furthermore, an additive effect of suppressing a non-specific reaction was observed by using the anti-L chain λ monoclonal antibody and the anti-L chain κ monoclonal antibody in combination.

### [Experimental Example 4: Examination of concentration of non-specific reaction suppressing agent in LTIA method: measurement of sIL-2R]

The concentration of sIL-2R in the sample was measured using a reagent according to the LTIA method, to which the anti-L chain λ monoclonal antibody and/or the anti-L chain κ monoclonal antibody according to the present invention was added at a low concentration, and the non-specific reaction suppressing effect was confirmed.

As a sample, the control samples (samples 1 to 6) used in Experimental Example 1 and some of the discrepant samples (samples 7, 8, and 10) were used. The confirmation of the effect was performed by comparing with results of Comparative Example 1 of Experimental Example 1.

### [Example 9] (Measurement by LTIA method: addition of anti-L chain λ monoclonal antibody)

The measurement was performed by the same method as in Comparative Example 1, except that the anti-L chain λ monoclonal antibody (S06215 antibody prepared in Reference Example 1) was added to the first reagent shown in Comparative Example 1 such that the concentration of the antibody was 20 µg/mL. Measurement results were shown in Table 5.

### [Example 10] (Measurement by LTIA method: addition of anti-L chain λ monoclonal antibody)

The measurement was performed by the same method as in Comparative Example 1, except that the anti-L chain λ monoclonal antibody (S06215 antibody prepared in Reference Example 1) was added to the first reagent shown in Comparative Example 1 such that the concentration of the antibody was 50 µg/mL. Measurement results were shown in Table 5.

### [Example 11] (Measurement by LTIA method: addition of anti-L chain κ monoclonal antibody)

The measurement was performed by the same method as in Comparative Example 1, except that the anti-L chain κ monoclonal antibody (S06205 antibody prepared in Reference Example 1) was added to the first reagent shown in Comparative Example 1 such that the concentration of the antibody was 20 µg/mL. Measurement results were shown in Table 5.

### [Example 12] (Measurement by LTIA method: addition of anti-L chain κ monoclonal antibody)

The measurement was performed by the same method as in Comparative Example 1, except that the anti-L chain κ monoclonal antibody (S06205 antibody prepared in Reference Example 1) was added to the first reagent shown in Comparative Example 1 such that the concentration of the antibody was 50 µg/mL. Measurement results were shown in Table 5.

### [Example 13] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 1 such that the concentration of each antibody was 20 µg/mL. Measurement results were shown in Table 5.

### [Example 14] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 1 such that the concentration of each antibody was 50 µg/mL. Measurement results were shown in Table 5.

### <Results and Considerations>

From the results of Comparative Example 1 and Examples 9 to 14, the effect of the non-specific reaction suppressing agent according to the present invention on the low concentration side was considered.

### (1) Measurement results of control samples (sample numbers 1 to 6)

All of the examples were generally the same as Comparative Example 1, which did not include the non-specific reaction suppressing agent. Here, from the results of Experimental Example 1, since Comparative Example 1 is equivalent to the measured value of the CLEIA method (Control Example 1), it can be determined that all of the examples are equivalent to Control Example 1. Therefore, it was found that the addition of the anti-L chain λ monoclonal antibody or the anti-L chain κ monoclonal antibody according to the present invention does not affect the measured value of the control sample in a range of 20 to 50 µg/mL.

### (2) Measurement results of discrepant samples (sample numbers 7, 8, and 10)

As described above, in the sample numbers 7, 8, and 10, the measured values of the LTIA method to which the non-specific reaction suppressing agent was not added (Comparative Example 1) were each 1814 U/mL, 738 U/mL, and 1857 U/mL, which deviated from the measured values of Control Example 1.

On the other hand, for the sample number 7, the measured values of the LTIA method to which the anti-L chain λ monoclonal antibody according to the present invention were added (Examples 9 and 10) such that the concentrations of the antibody was each 20 µg/mL and 50 µg/mL, were each 1262 U/mL and 812 U/mL, which were lower than those of Comparative Example 1.

The measured value of the LTIA method in which 20 µg/mL anti-L chain λ monoclonal antibody and 20 µg/mL anti-L chain κ monoclonal antibody were used in combination (Example 13) and the measured value of the LTIA method in which 50 µg/mL anti-L chain λ monoclonal antibody and 50 µg/mL anti-L chain κ monoclonal antibody were used in combination (Example 14) were each 1235 U/mL and 779 U/mL, which were lower than those in Comparative Example 1.

In addition, for the sample number 10, the measured values of the LTIA method to which the anti-L chain κ monoclonal antibody according to the present invention were added (Examples 11 and 12) such that the concentrations of the antibody was each 20 µg/mL and 50 µg/mL, were each 1777 U/mL and 1674 U/mL, which were lower than those of Comparative Example 1.

The measured value of the LTIA method in which 20 µg/mL anti-L chain λ monoclonal antibody and 20 µg/mL anti-L chain κ monoclonal antibody were used in combination (Example 13) and the measured value of the LTIA method in which 50 µg/mL anti-L chain λ monoclonal antibody and 50 µg/mL anti-L chain κ monoclonal antibody were used in combination (Example 14) were each 1796 U/mL and 1685 U/mL, which were lower than those in Comparative Example 1.

(3) As described above, in the immunoassay method, it was found that even in a case where the anti-L chain λ monoclonal antibody or the anti-L chain κ monoclonal antibody according to the present invention was present in the first reagent at a concentration of 20 to 50 µg/mL and the weight of the anti-L chain λ monoclonal antibody or the anti-L chain κ monoclonal antibody was 4.3 to 10.7 µg with respect to 10 µL of the sample, it was possible to suppress a non-specific reaction derived from the sample. In addition, in a case where the anti-L chain λ monoclonal antibody and the anti-L chain κ monoclonal antibody were used in combination at the above-described concentration, it was also possible to suppress various non-specific reactions derived from the sample.

**[Table 5]**

| Measurement results of sIL-2R (U/mL) | | | | | | |
|---|---|---|---|---|---|---|
| | | | Example 9 | Example 10 | Example 11 | Control Example 1 |
| Measuring method | | | LTIA method | LTIA method | LTIA method | CLEIA method |
| Additive and addition concentration | | | Anti-L chain λ monoclonal antibody 20 µg/mL | Anti-L chain λ monoclonal antibody 50 µg/mL | Anti-L chain κ monoclonal antibody 20 µg/mL | - |
| Control sample | Sample number | 1 | 272 | 264 | 267 | 296 |
| | | 2 | 581 | 586 | 576 | 598 |
| | | 3 | 437 | 455 | 447 | 438 |
| | | 4 | 689 | 702 | 723 | 670 |
| | | 5 | 919 | 928 | 931 | 906 |
| | | 6 | 2026 | 2026 | 2048 | 2140 |
| Discrepant sample | Sample number | 7 | 1262 | 812 | 1808 | 672 |
| | | 8 | 668 | 641 | 733 | 601 |
| | | 10 | 1873 | 1861 | 1777 | 1640 |
| | | | Example 12 | Example 13 | Example 14 | Comparative Example 2 |
| Measuring method | | | LTIA method | LTIA method | LTIA method | LTIA method |
| Additive and addition concentration | | | Anti-L chain κ monoclonal antibody 50 µg/mL | Anti-L chain λ monoclonal antibody 20 µg/mL | Anti-L chain λ monoclonal antibody 50 µg/mL | No additive |
| | | | | Anti-L chain κ monoclonal antibody 20 µg/mL | Anti-L chain κ monoclonal antibody 50 µg/mL | |
| Control sample | Sample number | 1 | 266 | 264 | 256 | 288 |
| | | 2 | 590 | 575 | 589 | 600 |
| | | 3 | 452 | 415 | 449 | 438 |
| | | 4 | 715 | 700 | 697 | 708 |
| | | 5 | 954 | 929 | 935 | 915 |
| | | 6 | 2041 | 2054 | 2041 | 2038 |
| Discrepant sample | Sample number | 7 | 1756 | 1235 | 779 | 1814 |
| | | 8 | 717 | 668 | 626 | 748 |
| | | 10 | 1674 | 1796 | 1685 | 1857 |

### [Experimental Example 5: Examination of concentration of non-specific reaction suppressing agent in LTIA method: measurement of sIL-2R]

The concentration of sIL-2R in the sample was measured using a reagent according to the LTIA method, to which the anti-L chain λ monoclonal antibody and/or the anti-L chain κ monoclonal antibody according to the present invention was added, and the non-specific reaction suppressing effect was confirmed.

As a sample, a sample (control sample: samples 31 to 33) showing a value close to the measured value by the chemiluminescent enzyme immunoassay method (CLEIA method) (Control Example 1) and a sample (discrepant sample: samples 34 to 37) exhibiting a non-specific reaction and significantly deviating from the measured value of Control Example 1 were used.

### [Comparative Example 7] (Measurement by LTIA method: addition of anti-human IgG, IgA, and IgM polyclonal antibodies)

The measurement was performed by the same method as in Comparative Example 1, except that an anti-human IgG polyclonal antibody, an anti-human IgA polyclonal antibody, and an anti-human IgM polyclonal antibody (manufactured by SeraCare Life Sciences, Inc. S), which was commercially available, were added to the first reagent shown in Comparative Example 1 such that the concentration of each antibody was 400 µg/mL. Measurement results were shown in Table 6.

### [Example 15] (Measurement by LTIA method: addition of anti-L chain κ monoclonal antibody)

The measurement was performed by the same method as in Comparative Example 1, except that the anti-L chain κ monoclonal antibody (S06215 antibody prepared in Reference Example 1) was added to the first reagent shown in Comparative Example 1 such that the concentration of the antibody was 400 µg/mL. Measurement results were shown in Table 6.

### [Example 12] (Measurement by LTIA method: addition of anti-L chain κ monoclonal antibody)

The measurement was performed by the same method as in Comparative Example 1, except that the anti-L chain κ monoclonal antibody (S06205 antibody prepared in Reference Example 1) was added to the first reagent shown in Comparative Example 1 such that the concentration of the antibody was 50 µg/mL. Measurement results were shown in Table 6. In addition, "ND" in Table 6 means that there is no data (No Data).

### [Example 2] (Measurement by LTIA method: addition of anti-L chain κ monoclonal antibody)

The measurement was performed by the same method as in Comparative Example 1, except that the anti-L chain κ monoclonal antibody (S06205 antibody prepared in Reference Example 1) was added to the first reagent shown in Comparative Example 1 such that the concentration of the antibody was 200 µg/mL. Measurement results were shown in Table 6.

### [Example 16] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody) and an anti-L chain κ monoclonal antibody (S06205 antibody prepared in Reference Example 1) were added to the first reagent shown in Comparative Example 1 such that the concentration of each antibody was 400 µg/mL. Measurement results were shown in Table 6.

### [Example 14] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 1 such that the concentration of each antibody was 50 µg/mL. Measurement results were shown in Table 6. In addition, "ND" in Table 6 means that there is no data (No Data).

### [Example 3] (Measurement by LTIA method: addition of anti-L chain λ monoclonal antibody and anti-L chain κ monoclonal antibody)

The measurement was performed by the same method as in Comparative Example 1, except that an anti-L chain λ monoclonal antibody (S06215 antibody prepared in Reference Example 1) and an anti-L chain κ monoclonal antibody (S06205 antibody prepared in Reference Example 1) were added to the first reagent shown in Comparative Example 1 such that the concentration of each antibody was 200 µg/mL. Measurement results were shown in Table 6.

**[Table 6]**

| Measurement results of sIL-2R (U/mL) | | | | | | | |
|---|---|---|---|---|---|---|---|
| | | | Example 12 | Example 2 | Example 15 | Control Example 1 | Comparative Example 1 |
| Measuring method | | | LTIA method | LTIA method | LTIA method | CLEIA method | LTIA method |
| Additive and addition concentration | | | Anti-L chain λ monoclonal antibody 50 µg/mL | Anti-L chain λ monoclonal antibody 200 µg/mL | Anti-L chain κ monoclonal antibody 400 µg/mL | - | No additive |
| Control sample | Sample number | 31 | 332 | 343 | 351 | 312 | 342 |
| | | 32 | 2568 | 2596 | 2571 | 2530 | 2537 |
| | | 33 | 3156 | 3213 | 3208 | 3180 | 3104 |
| Discrepant sample | Sample number | 34 | 419 | 372 | 350 | 306 | 504 |
| | | 35 | 480 | 447 | 470 | 328 | 632 |
| | | 36 | ND | 468 | 449 | 315 | 577 |
| | | 37 | ND | 345 | 291 | 200 | 506 |
| | | | Example 14 | Example 3 | Example 16 | Comparative Example 7 | |
| Measuring method | | | LTIA method | LTIA method | LTIA method | LTIA method | |
| Additive and addition concentration | | | Anti-L chain λ monoclonal antibody 50 µg/mL | Anti-L chain λ monoclonal antibody 200 µg/mL | Anti-L chain λ monoclonal antibody 400 µg/mL | Anti-human IgG, IgA, or IgM polyclonal antibody 400 µg/mL | |
| | | | Anti-L chain κ monoclonal antibody 50 µg/mL | Anti-L chain κ monoclonal antibody 200 µg/mL | Anti-L chain κ monoclonal antibody 400 µg/mL | | |
| Control sample | Sample number | 31 | 358 | 340 | 356 | 376 | |
| | | 32 | 2525 | 2561 | 2599 | 2402 | |
| | | 33 | 3212 | 3237 | 3220 | 3168 | |
| Discrepant sample | Sample number | 34 | 434 | 369 | 410 | 464 | |
| | | 35 | 438 | 398 | 424 | 502 | |
| | | 36 | ND | 452 | 472 | 508 | |
| | | 37 | ND | 324 | 297 | 413 | |

### <Results and Considerations>

From the results of Control Example 1, Comparative Examples 1 and 7, Examples 2, 3, and 14 to 16, the effect of the non-specific reaction suppressing agent according to the present invention was considered.

### (1) Measurement results of control samples (sample numbers 31 to 33)

All of the examples were generally the same as Comparative Example 1, which did not include the non-specific reaction suppressing agent. Here, from the results of Experimental Example 1, since Comparative Example 1 is equivalent to the measured value of the CLEIA method (Control Example 1), it can be determined that all of the examples are equivalent to Control Example 1. In addition, from the results of Experimental Example 4, it was found that the measurement value of the control sample was not affected in a range of 20 to 50 µg/mL. Therefore, it was found that the addition of the anti-L chain λ monoclonal antibody or the anti-L chain κ monoclonal antibody according to the present invention does not affect the measured value of the control sample in a range of 20 to 400 µg/mL.

### (2) Measurement results of discrepant samples (sample numbers 34 to 37)

In the sample numbers 34 to 37, the measured values of the LTIA method to which the non-specific reaction suppressing agent was not added (Comparative Example 1) were deviated from the measured values of Control Example 1 as described above.

In the sample number 34, the measured value of the LTIA method to which 400 µg/mL of the anti-L chain κ monoclonal antibody according to the present invention was added (Example 15) was 350 U/mL, which showed a tendency to approach Control Example 1 as compared with Comparative Example 1. Furthermore, the measured values of the LTIA method in which 50 µg/mL anti-L chain λ monoclonal antibody and 50 µg/mL anti-L chain κ monoclonal antibody were used in combination (Example 14), the measured values of the LTIA method in which 200 µg/mL anti-L chain λ monoclonal antibody and 200 µg/mL anti-L chain κ monoclonal antibody were used in combination (Example 3), and the measured values of the LTIA method in which 400 µg/mL anti-L chain λ monoclonal antibody and 400 µg/mL anti-L chain κ monoclonal antibody were used in combination (Example 16) were each 434 U/mL, 369 U/mL, and 410 U/mL, which showed a tendency to approach Control Example 1 as compared with Comparative Example 1. The same tendency was observed in other samples.

In addition, in the sample number 34, the measured values of the LTIA method in which the anti-L chain κ monoclonal antibody according to the present invention was added such that the concentrations of the antibody were each 50 µg/mL, 200 µg/mL, or 400 µg/mL (Example 12, Example 2, and Example 15) were each 419 U/mL, 372 U/mL, and 350 U/mL, and a remarkable non-specific reaction suppressing effect was exhibited at an addition concentration lower than that of the anti-human IgG polyclonal antibody, the anti-human IgA polyclonal antibody, and the anti-human IgM polyclonal antibody (Comparative Example 7). In addition, the measured values of the LTIA method in which 50 µg/mL anti-L chain λ monoclonal antibody and 50 µg/mL anti-L chain κ monoclonal antibody were used in combination (Example 14), the measured values of the LTIA method in which 200 µg/mL anti-L chain λ monoclonal antibody and 200 µg/mL anti-L chain κ monoclonal antibody were used in combination (Example 3), and the measured values of the LTIA method in which 400 µg/mL anti-L chain λ monoclonal antibody and 400 µg/mL anti-L chain κ monoclonal antibody were used in combination (Example 16) were each 434 U/mL, 369 U/mL, and 410 U/mL, and a remarkable non-specific reaction suppressing effect was exhibited at an addition concentration lower than that of the anti-human IgG polyclonal antibody, the anti-human IgA polyclonal antibody, and the anti-human IgM polyclonal antibody (Comparative Example 7). The same tendency was observed in other samples.

(3) As described above, in the immunoassay method, it was found that even in a case where the anti-L chain λ monoclonal antibody or the anti-L chain κ monoclonal antibody according to the present invention was present in the first reagent at a concentration of 20 to 400 µg/mL and the weights of the anti-L chain λ monoclonal antibody or the anti-L chain κ monoclonal antibody was 4.3 to 85.7 µg with respect to 10 µL of the sample, it was possible to suppress a non-specific reaction derived from the sample. In addition, in a case where the anti-L chain λ monoclonal antibody and the anti-L chain κ monoclonal antibody were used in combination at the above-described concentration, it was also possible to suppress various non-specific reactions derived from the sample.

In addition, according to the present invention, it was possible to suppress the non-specific reaction even at a lower addition concentration as compared with the anti-human IgG polyclonal antibody, the anti-human IgA polyclonal antibody, and the anti-human IgM polyclonal antibody, which are commercially available non-specific reaction suppressing agents.

### [Experimental Example 6: Examination of concentration of non-specific reaction suppressing agent in ELISA method: measurement of sIL-2R]

The concentration of sIL-2R in the sample was measured using a reagent according to the ELISA method, to which the anti-L chain λ monoclonal antibody and/or the anti-L chain κ monoclonal antibody according to the present invention was added at a low concentration, and the non-specific reaction suppressing effect was confirmed.

As a sample, a sample (control sample: samples 31 and 33) showing a value close to the measured value by the chemiluminescent enzyme immunoassay method (CLEIA method) (Control Example 1) and a sample (discrepant sample: samples 38 to 40) exhibiting a non-specific reaction and deviating from the measured value of Control Example 1 were used.

### <Method>

(1) The anti-sIL-2R monoclonal antibody (clone number of 92212) was dissolved in a 20 mM phosphate buffer solution (pH 7.2; hereinafter, referred to as PBS) containing 150 mM sodium chloride to a concentration of 10 µg/mL, 100 µL of the dissolved solution was dispensed into each well of a 96-well microplate, and the dissolved solution was allowed to stand at 4°C overnight.
(2) Each of the wells were washed three times with 400 µL of PBS containing 0.05% Tween (registered trademark) 20 (hereinafter, referred to as PBST), and then 200 µL of PBST containing 1% bovine serum albumin (hereinafter, referred to as BSA-PBST) was added thereto, and blocking was performed at room temperature for 1 hour. This was used as a plate for ELISA.
(3) Each well of the ELISA plate was washed three times with 400 µL of PBST, then 100 µL of a sample diluted 40-fold with a sample diluent liquid to which components described later were added to BSA-PBST was added to each well, and the mixture was allowed to stand at room temperature for 1 hour.
(4) Each well was washed three times with 400 µL of PBST, then 100 µL of a biotin-labeled anti-sIL-2R monoclonal antibody (clone number of 92204R) diluted to 0.50 µg/mL with BSA-PBST was dispensed into each well, and the mixture was allowed to stand at room temperature for 1 hour.
(5) Each well was washed three times with 400 µL of PBST, then 100 µL of HRP-labeled streptavidin (Thermo Fisher Scientific, Inc.) diluted to 0.20 µg/mL with BSA-PBST was dispensed into each well, and the mixture was allowed to stand at room temperature for 30 minutes.
(6) The wells were washed three times with 400 µL of PBST, 50 µL of a citrate buffer solution (pH 5.0) containing 0.2% ortho-phenylenediamine and 0.02% hydrogen peroxide was added thereto, the mixture was allowed to stand at room temperature for 10 minutes, 50 µL of 4.5 N sulfuric acid was added thereto to stop the enzymatic reaction, and the absorbance at a wavelength of 492 nm was measured.
(7) A standard substance having a known concentration was used as a calibrator, and the sIL-2R value in the test sample was calculated.
(8) A calibration curve for each test using each reagent was created using the measurement results of the calibrator. The measured value of each sample was obtained using the obtained calibration curve. A value obtained by dividing the measured value in Comparative Example 9 and 10 and Example 17 for each sample by the measured value in Control Example 1 was calculated and evaluated as a relative ratio (%). In a case where the relative ratio was 90% or more and 110% or less, it was determined that the measurement value was not deviated from the measurement value of Control Example 1.

### [Comparative Example 9] (Measurement by ELISA method: without additive)

The measurement was performed using BSA-PBST as a sample diluent liquid. The results of the measured values are shown in Table 7, and the results of the relative ratios are shown in Table 8.

### [Comparative Example 10] (Measurement by ELISA method: addition of HBR-1)

The measurement was performed by the same method as in Comparative Example 9, except that HBR-1 (manufactured by SCANTIBODIES LABORATORY, INC.), a commercially available non-specific reaction suppressing agent, was added to the sample diluent liquid shown in Comparative Example 9 such that the concentration of the HBR-1 was 400 µg/mL. The results of the measured values are shown in Table 7, and the results of the relative ratios are shown in Table 8.

### [Example 17] (Measurement by ELISA method: addition of anti-L chain λ monoclonal antibody and anti-L chain κ monoclonal antibody)

The measurement was performed by the same method as in Comparative Example 9, except that an anti-L chain λ monoclonal antibody (S06215 antibody prepared in Reference Example 1) and an anti-L chain κ monoclonal antibody (S06205 antibody prepared in Reference Example 1) were added to the sample diluent liquid shown in Comparative Example 9 such that the concentration of each antibody was 200 µg/mL. The results of the measured values are shown in Table 7, and the results of the relative ratio with respect to Target Example 1 are shown in Table 8.

**[Table 7]**

| Measurement results of sIL-2R (U/mL) | | | | | | |
|---|---|---|---|---|---|---|
| | | | Example 17 | Comparative Example 9 | Comparative Example 10 | Control Example 1 |
| Measuring method | | | ELISA method | ELISA method | ELISA method | CLEIA method |
| Additive and addition concentration | | | Anti-L chain λ monoclonal antibody 200 µg/mL | No additive | HBR-1 400 µg/mL | - |
| | | | Anti-L chain κ monoclonal antibody 200 µg/mL | | | |
| Control sample | Sample number | 31 | 337 | 325 | 319 | 312 |
| | | 33 | 3265 | 3137 | 3148 | 3180 |
| Discrepant sample | Sample number | 38 | 884 | 991 | 1088 | 903 |
| | | 39 | 755 | 831 | 799 | 724 |
| | | 40 | 1408 | 1503 | 1586 | 1330 |

**[Table 8]**

| Measurement results of sIL-2R (%) | | | | | | |
|---|---|---|---|---|---|---|
| | | | Example 17 | Comparative Example 9 | Comparative Example 10 | Control Example 1 |
| Measuring method | | | ELISA method | ELISA method | ELISA method | CLEIA method |
| Additive and addition concentration | | | Anti-L chain λ monoclonal antibody 200 µg/mL | No additive | HBR-1 400 µg/mL | - |
| | | | Anti-L chain κ monoclonal antibody 200 µg/mL | | | |
| Control sample | Sample number | 31 | 108 | 104 | 102 | - |
| | | 33 | 103 | 99 | 99 | - |
| Discrepant sample | Sample number | 38 | 98 | 110 | 120 | |
| | | 39 | 104 | 115 | 110 | - |
| | | 40 | 106 | 113 | 119 | - |

### <Results and Considerations>

From the results of Control Example 1, Comparative Examples 9 and 10, Example 17, the effect of the non-specific reaction suppressing agent according to the present invention was considered. The control samples 31 and 33 in which the proportion (relative ratio) of the measured value of the ELISA method to which the additive was not added (Comparative Example 9) to the measured value of the CLEIA method (Control Example 1) was 99% to 104% were evaluated as control samples in which a non-specific reaction did not occur in the ELISA method. In addition, the discrepant samples 38 to 40 in which the proportion (relative ratio) of the measured value of the ELISA method to which the additive was not added (Comparative Example 9) to the measured value of the CLEIA method (Control Example 1) was 110% to 115% were evaluated as discrepant samples in which a non-specific reaction occured in the ELISA method.

### (1) Measurement results of control samples (sample numbers 31 and 33)

All of the examples were generally the same as Comparative Example 9, which did not include the non-specific reaction suppressing agent, and Control Example 1. Therefore, it was found that the addition of the anti-L chain λ monoclonal antibody or the anti-L chain κ monoclonal antibody according to the present invention (200 µg/mL each) does not affect the measured value of the control sample.

### (2) Measurement results of discrepant samples (sample numbers 38 to 40)

In the sample numbers 38 to 40, the measured values of the ELISA method to which the non-specific reaction suppressing agent was not added (Comparative Example 9) were deviated from the measured values of Control Example 1.

For the sample number 38, the measured value of the ELISA method to which HBR-1 was added such that the concentration of HBR-1 was 400 µg/mL (Comparative Example 10) was 1088 U/mL, and the non-specific suppressing effect was insufficient.

The measured value of the ELISA method in which 200 µg/mL anti-L chain λ monoclonal antibody and 200 µg/mL anti-L chain κ monoclonal antibody according to the present invention were used in combination (Example 17) was 884 U/mL, which was a lower numerical value than that in Comparative Example 9. The same tendency was obtained for other discrepant samples. Even in the ELISA reagent which was a measuring method having B/F separation, in a case where the component according to the present invention was added to the sample diluent liquid, the consistency with the measured value of the CLEIA method which was a measuring method having B/F separation was improved.

The non-specific reaction that could not be suppressed even by HBR-1, which is a commercially available non-specific reaction suppressing agent, could be suppressed by the present invention, the non-specific reaction suppressing effect by the anti-L chain λ monoclonal antibody or the anti-L chain κ monoclonal antibody according to the present invention was a result which was more than expected.

(3) As described above, even in the ELISA reagent which was a measuring method having B/F separation, in a case where the component according to the present invention was added to the sample diluent liquid, the consistency with the measured value of the CLEIA method was improved.

In general, in a case where B/F separation occurred, a non-specific reaction was less likely to occur, but there was a possibility that the non-specific reaction that had occurred to a small extent could be suppressed by the present component.

### INDUSTRIAL APPLICABILITY

According to the present invention, it became possible to realize an accurate immunoassay method in which a non-specific reaction that cannot be resolved by non-specific reaction suppressing agents in the related art even in a case where a non-specific factor is contained in a sample can be suppressed by performing an immunoreaction in the presence of both or either of the anti-L chain λ monoclonal antibody and the anti-L chain κ monoclonal antibody.

## Claims

1. An immunoassay method that is an immunologically measuring method of a target substance to be measured in a sample, the immunoassay method comprising:
performing an immunoreaction in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody,
provided that an anti-immunoglobulin L chain lambda antibody and an anti-immunoglobulin L chain kappa antibody is excluded from the target substance to be measured.

2. The immunoassay method according to Claim 1,
wherein the immunoassay method is an immunoassay method based on a homogeneous method or an immunoassay method based on a heterogeneous method.

3. The immunoassay method according to Claim 2, comprising:
a step of bringing a sample containing the target substance to be measured into contact with the anti-immunoglobulin L chain lambda monoclonal antibody or the anti-immunoglobulin L chain kappa monoclonal antibody;
a step of adding a specific binding partner for the target substance to be measured; and
a step of detecting a signal derived from a reaction between the target substance to be measured and the specific binding partner in the solution.

4. The immunoassay method according to Claim 2,
wherein the immunoassay method based on a homogeneous method is a latex turbidimetric immunoassay method.

5. The immunoassay method according to Claim 4, comprising:
a step of bringing a sample containing the target substance to be measured into contact with the anti-immunoglobulin L chain lambda monoclonal antibody or the anti-immunoglobulin L chain kappa monoclonal antibody in a solution;
a step of adding latex particles carrying a specific binding partner for the target substance to be measured to the solution; and
a step of optically detecting a degree of aggregation of the latex particles in the solution.

6. A non-specific reaction suppression method in an immunologically measuring method of a target substance to be measured in a sample, the non-specific reaction suppression method comprising:
performing an immunoreaction in a presence of an anti-immunoglobulin L chain lambda monoclonal antibody and an anti-immunoglobulin L chain kappa monoclonal antibody, in a presence of the anti-immunoglobulin L chain lambda monoclonal antibody, or in a presence of the anti-immunoglobulin L chain kappa monoclonal antibody.

7. The non-specific reaction suppression method according to Claim 6,
wherein the immunologically measuring method is an immunoassay method based on a homogeneous method or an immunoassay method based on a heterogeneous method.

8. The non-specific reaction suppression method according to Claim 7,
wherein the immunoassay method based on a homogeneous method is a latex turbidimetric immunoassay method.

9. The non-specific reaction suppression method according to Claim 8, comprising:
a step of bringing a sample containing the target substance to be measured into contact with the anti-immunoglobulin L chain lambda monoclonal antibody or the anti-immunoglobulin L chain kappa monoclonal antibody in a solution;
a step of adding latex particles carrying a specific binding partner for the target substance to be measured to the solution; and
a step of optically detecting a degree of aggregation of the latex particles in the solution.

10. An immunoassay reagent comprising:
both or either of an anti-immunoglobulin L chain lambda monoclonal antibody or an anti-immunoglobulin L chain kappa monoclonal antibody.

11. The immunoassay reagent according to Claim 10,
wherein the immunoassay reagent is for an immunoassay method based on a homogeneous method or an immunoassay method based on a heterogeneous method.

12. The immunoassay reagent according to Claim 11,
wherein the immunoassay method based on a homogeneous method is a latex turbidimetric immunoassay method.

13. An immunoassay reagent kit comprising:
both or either of an anti-immunoglobulin L chain lambda monoclonal antibody or an anti-immunoglobulin L chain kappa monoclonal antibody.

14. The immunoassay reagent kit according to Claim 13,
wherein the immunoassay reagent is for an immunoassay method based on a homogeneous method or an immunoassay method based on a heterogeneous method.

15. The immunoassay reagent kit according to Claim 14,
wherein the immunoassay method based on a homogeneous method is a latex turbidimetric immunoassay method.

16. The immunoassay reagent kit according to Claim 14,
wherein the method based on a homogeneous method is a latex turbidimetric immunoassay method, and
the immunoassay reagent kit includes
(1) a first reagent containing both or either of an anti-immunoglobulin L chain lambda monoclonal antibody or an anti-immunoglobulin L chain kappa monoclonal antibody, and
(2) a second reagent containing latex particles carrying a specific binding partner for a target substance to be measured.

17. A composition comprising:
both or either of an anti-immunoglobulin L chain lambda monoclonal antibody or an anti-immunoglobulin L chain kappa monoclonal antibody.

18. A non-specific reaction suppressing agent in an immunoassay method, comprising, as an effective component:
both or either of an anti-immunoglobulin L chain lambda monoclonal antibody or an anti-immunoglobulin L chain kappa monoclonal antibody.

19. Use of the composition according to Claim 17 as a non-specific reaction suppressing agent in an immunoassay method.
